# EUROPEAN PATENT SPECIFICATION

(11) **EP 1 571 158 B1**
(45) Date of publication and mention of the grant of the patent: **14.10.2009**
(21) Application number: 04027742.8
(22) Date of filing: 04.08.2000
(51) Int. Cl.: C07K 14/47, A61K 38/04, G01N 33/68

(54) **Globular assembly of amyloid beta protein and uses thereof**
Globularer Aufbau vom Amyloid-beta- protein und deren Verwendungen
Assemblage de protéine amyloide beta globulaire et ses utilisations

(30) Priority: 04.08.1999 US 369236
(43) Date of publication of application: 07.09.2005
(62) Divisional of application: 00952571.8
(73) Proprietor: UNIVERSITY OF SOUTHERN CALIFORNIA, Los Angeles, CA 90089 (US); Northwestern University, Evanston Illinois 60208 (US)
(72) Inventor: Kraft, Grant A., Glenview, IL 60025 (US); Klein, William L., Winnetka, IL 60093 (US); Chromy, Brett A., Danvill, CA 94526 (US); Lambert, Mary P., Glenview, IL 60025 (US); Finch, Caleb E., Altadena, CA 91101 (US); Morgan, Todd, Manhattan Beach, CA 90266 (US); Wals, Pat, Nevada City, CA 90059-9686 (US); Rozovsky, Irina, Pasadena, CA 91107 (US); Barlow, Ann, Evanston, IL 60201 (US)
(74) Representative: Duffy, Assumpta Dympna

(56) References cited:
- WO-A-98/33815
- M P LAMBERT ET AL.: "Diffusible, nonfibrillar ligands derived from A-beta 1-42 are potent central nervous system neurotoxins" PROCEEDINGS OF THE NATIONAL ACADEMY OF SCIENCES OF USA., vol. 95, no. 11, 26 May 1998 (1998-05-26), pages 6448-6453, XP002160443 US NATIONAL ACADEMY OF SCIENCE, WASHINGTON, DC.
- YU-MIN KUO ET AL.: "Water soluble A-beta (N-40, N-42) oligomers in normal and Alzheimer disease brains" JOURNAL OF BIOLOGICAL CHEMISTRY., vol. 271, no. 8, 23 February 1998 (1998-02-23), pages 4077-4081, XP001068752 US AMERICAN SOCIETY OF BIOLOGICAL CHEMISTS, BALTIMORE, MD.

### SEQUENCE LISTING

(1) GENERAL INFORMATION:
   (i) APPLICANT:
      (A) NAME: Grant A. Krafft, William L. Klein, Brett A. Chromy, Mary P. Lambert, Caleb E. Finch, Todd Morgan, Pat wals, Irina Rozovsky, Ann Barlow
   (ii) TITLE OF INVENTION: Amyloid β Protein (Globular Assembly and Uses Thereof)
   (iii) NUMBER OF SEQUENCES: 4
   (iv) CORRESPONDENCE ADDRESS:
      (A) ADDRESSEE: McDonnell Boehnen Hulbert & Berghoff
      (B) STREET: 300 South wacker Drive
      (C) CITY: Chicago
      (D) STATE: IL
      (E) COUNTRY: USA
      (F) ZIP: 60606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (US)
   (vi) CURRENT APPLICATION DATA:
      (A) APPLICATION NUMBER: unassigned
      (B) FILING DATE: 04-AUG-1999
      (C) CLASSIFICATION DATA:
   (vii) PRIOR APPLICATION DATA:
      (A) APPLICATION NUMBER: US 08/796,089 (B) PILING DATE: O5-FEB-1997
(2) INFORMATION FOR SEQ ID NO:1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 1:
      GCACCTTCTT TCCCTTCATC 20
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 2:
      TGCTGATGTA CCAGTTGGGG 20
(2) INFORMATION FOR SEQ ID NO: 3:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 3:
      CAGTCCTTGA CCTGCGACC 19
(2) INFORMATION FOR SEQ ID NO: 4:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 4:
      GCCTCACATC ACATCCTTG 19

### SEQUENCE LISTING

(1) GENERAL INFORMATION:
   (i) APPLICANT:
      (A) NAME: Grant A. Krafft, William L. Klein, Brett A. Chromy, Mary P. Lambert, Caleb E. Finch, Todd Morgan, Pat Wals, Irina Rozovsky, Ann Barlow
   (ii) TITLE OF INVENTION: Amyloid β Protein (Globular Assembly and Uses Thereof)
   (iii) NUMBER OF SEQUENCES: 4
   (iv) CORRESPONDENCE ADDRESS:
      (A) ADDRESSEE: McDonnell Boehnen Hulbert & Berghoff
      (B) STREET: 300 South Wacker Drive
      (C) CITY: Chicago
      (D) STATE: IL
      (E) COUNTRY: USA
      (F) ZIP: 60606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (US)
   (vi) CURRENT APPLICATION DATA:
      (A) APPLICATION NUMBER: unassigned
      (B) FILING DATE: 04-AUG-1999
      (C) CLASSIFICATION DATA:
   (vii) PRIOR APPLICATION DATA:
      (A) APPLICATION NUMBER: US 08/796,089 (B) FILING DATE: 05-FEB-1997
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 1:
      GCACCTTCTT TCCCTTCATC 20
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 2:
      TGCTGATGTA CCAGTTGGGG 20
(2) INFORMATION FOR SEQ ID NO: 3:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 3:
      CAGTCCTTGA CCTGCGACC 19
(2) INFORMATION FOR SEQ ID NO: 4:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 4:
      GCCTCACATC ACATCCTTG 19

## Description

### TECHNICAL FIELD OF INVENTION

The present invention pertains to a new composition of matter, amyloid beta-derived dementing ligands (ADDLs). ADDLs comprise amyloid β peptide assembled into soluble globular non-fibrillar oligomeric structures that are capable of activating specific cellular processes. Described herein are compounds that block the formation or activity of ADDLs, and methods of identifying such compounds. ADDL formation and activity is relevant *inter alia* to learning and memory. Modulation of ADDL formation or activity thus can be employed according to the invention in the treatment of learning and memory disorders, as well as other diseases, disorders or conditions that are due to the effects of the ADDLs.

### BACKGROUND OF THE INVENTION

Alzheimer's disease is a progressive neurodegenerative disease, characterized by distinct pathologies, including neurofibrillary tangles, neuritic plaques, neuronal atrophy, dendritic pruning and neuronal death. From a historical perspective, definitive diagnosis of Alzheimer's disease always has relied upon identification of specific pathologic hallmarks, namely the neurofibrillary tangles which represent the collapsed cytoskeleton of dead and dying neurons, and neuritic plaques, which are extracellular deposits of various protein, lipid, carbohydrate and salt compounds, the primary protein component of which is a 39-43 residue peptide known as amyloid β.

From the standpoint of disease impact, however, it is the symptoms manifest in Alzheimer's disease, namely the loss of memory, the erosion of cognitive functions, and the significant changes in personality and behavior, which are most significant. Underlying these symptomatic changes are specific cellular mechanisms that cause nerve cells to malfunction, and eventually to degenerate and die. These cellular mechanisms undoubtedly operate within a background environment that variously affords some level of protection, or exerts contributing and exacerbating effects. The result is a very broad age/incidence distribution curve, with few clues from population studies that point to specific causes.

Molecular genetics represents one realm of study where a clear picture of familial Alzheimer's disease is emerging. As described in more detail below, it is now clear from studies identifying mutations in 3 different proteins, APP and the presenilins I and 2, that the final common pathway leading to Alzheimer's disease is the increased production of amyloid β 1-42 (as well as amyloid β 1-43), which occurs in all of these different familial AD mutations. This is particularly noteworthy, because ADDLs, the central focus of the invention described herein, only form as stable entities from this longer form of amyloid, and not from the more prevalent, shorter form Aβ 1-40.

Amyloid β in Alzheimer's Disease. In 1984, Glenner and Wong succeeded in isolating and identifying the cerebrovascular amyloid associated with Alzheimer's disease (Glenner et al., Biochem. Biophys. Res. Commun., 120, 885-890, 1984a). Subsequently, the same 39-43 residue peptides now known as amyloid β were identified as the major protein component of Alzheimer's disease neuritic plaques (Glenner et al., Biochem. Biophys. Res. Commun., 122, 1131-1135 1984b; Masters et al., EMBO J., 4, 2757-2764, 1985a; Masters et al., Proc. Natl. Acad Sci., 82, 4245-4249, 1985b). This was the first time a discrete molecule had been linked to Alzheimer's disease, a disease which to that point had been characterized only by neuroanatomy and neuropathology descriptions. Amyloid β also was identified as the plaque component in brains of Down's Syndrome individuals, (Glenner et al, Biochem. Biophys. Res. Commun., 122, 1131-1135, 1984b; Masters et al., EMBO J., 4, 2757-2764, 1985a; Masters et al., Proc. Natl. Acad. Sci., 82, 4245-4249, 1985b) leading to the suggestion that the gene encoding it might exist on chromosome 21. By 1987, a number of groups had used the amyloid β sequence information and molecular genetics techniques to validate that suggestion, identifying the gene for the amyloid precursor protein (APP) (Kang et al., Nature, 325, 733, 1987; Tanzi et al., Science, 235, 880-884, 1987).

The APP gene is a large, multi-exon gene that is differentially spliced into a number of APP's (reviewed in Selkoe, In, Annual Review of Neuroscience, Cowan (Ed.), 17, ix + 623 p, 489-517, 1994). The proteins are large transmembrane proteins, now known to be processed by several pathways, one or more of which may generate amyloid β. The earliest studies of APP processing had suggested that amyloid β formation was not a normal process (Esch et al., Science, 248, 1122-1124 1990; Sisodia et al., Science, 248, 492-495, 1990), though subsequent studies in cultured cells and analysis of serum and cerebrospinal fluid have shown that amyloid β formation occurs as a normal process in many cell types, though its formation may not represent a predominant overall pathway.

Pivotal genetic studies of DNA from individuals afflicted with early onset of familial Alzheimer's disease revealed that mutations in a single gene, this same APP gene, were causative for this very severe form of the disease. Interestingly, several different mutations in the APP gene were found including three different single residue substitutions at Val 717, four residues downstream of the amyloid β1-42 C-terminus (Goate et al., Nature, 349, 704-6 1991; Chartier-Harlan et al., Nature, 353, 844-6 1991; Murrell et al., Science, 254, 97-9, 1991), and a two residue mutation (670-671) immediately upstream of the amyloid β N-terminus, associated with early onset familial Alzheimer's disease in a Swedish family (Mullan et al., Nature Genetics 1,345-347, 1992). When a vector encoding the cDNA of the Swedish mutant APP gene was transfected into cell lines to evaluate APP processing, it was found that six-eight times more amyloid β was formed, when compared with levels from wild-type APP (Citron et al., Nature, 360, 672-674, 1992; Cai et al., Science, 259, 514-516, 1993). It was also demonstrated that brain tissue extracts containing native human brain protease activities were able to process a fluorogenic octapeptide substrate encompassing the Swedish mutation more than 100-fold faster than the corresponding substrate based on the wild-type sequence (Ladror et al., J. Biol. Chem., 269, 18422-8, 1994). These results suggest that the mechanism by which the Swedish mutation causes early onset familial Alzheimer's disease involves substantial overproduction of amyloid β. Similar studies of amyloid formation in cells transfected with the 717 mutant APP also had been conducted, but the levels of amyloid β produced were not different from levels produced by wild-type APP. This led to mechanistic speculations that something other than amyloid β production was pathogenic for these mutations. A closer evaluation of processing of the APP 717 mutant, and the Swedish mutant APP by Younkin and co-workers (Suzuki et al., Science, 264, 1336-1340, 1994) provided a unified picture of these genetic Alzheimer's disease cases. In this study, not only were total levels of amyloid β production evaluated, but the specific lengths of the amyloid β peptides produced were also analyzed. The results confirmed that the 717 mutation led to more than a doubling of the ratio of amyloid β 1-42 to amyloid β 1-40 (a highly soluble peptide under physiologic conditions) even though total amyloid β levels did not change. The recently discovered presenilin 1 and 2 familial Alzheimer's disease mutations in genes residing on chromosome 14 (Sherrington et al., Nature, 375, 754-758, 1995) and chromosome 1 (Levy-Lahad et al., Science, 269, 970-973, 1995), respectively, have also been linked to significant overproduction of amyloid β 1-42. (Mann et al., Annals of Neurology, 40, 149-56, 1996; Schuener et al., Nature Medicine, 2, 864-70, 1996). Based on these findings, it appears that the pathogenic process mediated by these distinctly different familial Alzheimer's disease mutations is the production of greater levels of amyloid β1-42. This is the form of amyloid that aggregates most readily (Snyder et al., Biophys. J., 67,1216-28, 1994), that seeds aggregation of amyloid β to form neuritic plaques (Roher et al., Neurochem., 61, 1916-1926, 1993; Tamaoka et al., Biochem. Biophs. Res. Commun., 205, 834-842, 1994), and, as described herein, the form which unexpectedly forms stable higher order assemblies termed "ADDLs". WO 98/33815 discloses ADDLs comprising from 3 to 12 amyloid beta 1-42 (A β 1-42) monomers.

Non-amyloid Plaque Components in Alzheimer's Disease. Amyloid β is the major protein component of plaques, comprising more than 70% of the total protein. A variety of other protein components also are present, however, including α1-antichymotrypsin (ACT), heparin sulfate proteoglycans (HSPG), apolipoproteins E and J, butyrylcholinesterase (BChE), S-100B, and several complement components. While the importance of these components in the onset and progression of Alzheimer's disease has not been established, involvement of apo E isoforms in the disease has been established by genetic studies of Roses and colleagues (Strittmatter et al., Proc. Natl. Acad. Sci. USA, 90,1977-81, 1993), who discovered that a polymorphism in the apolipoprotein E gene, namely apo E4, correlated with earlier onset of Alzheimer's disease in a large set of late-onset familial Alzheimer's disease cases. Subsequent studies have confirmed that groups of individuals with apo E4 have a significantly greater risk of Alzheimer's disease and that the onset of Alzheimer's disease roughly parallels the gene dosage for apo E4. On a mechanistic level, studies have revealed that apo E4 binds with lower affinity to amyloid β than apo E3 or apo E2, isoforms which are associated with later onset of Alzheimer's disease. It has been suggested that these isoforms may exert a protective effect by more effective clearance of amyloid β 1-42 deposits (Ladu et al., J. Biol. Chem., 269, 23403-23406, 1994; Ladu et al., J. Biol. Chem., 270, 9039-42, 1995).

The role of other plaque components is not as clear, though recent studies (Oda et al., Exptl. Neurology, 136, 22-31, 1995) have shown that apo J (clusterin) can significantly enhance the toxicity of aggregated amyloid β 1-42 *in vitro.* It also has been reported that HSPG enhances the toxicity of amyloid β 1-40 when injected into rat brain (Snow et al., Soc. Neurosci. Abstr., 18, 1465, 1992). Wright et al. (Ann Neurol., 34, 373-384, 1993) demonstrated that amyloid plaques from Alzheimer's disease brain contain significant levels of BChE, while amyloid plaques from elderly non-demented individuals do not. The acute phase inflammatory protein ACT also is upregulated in Alzheimer's disease brain, and it is known to associate with the N-terminal 16 residues of amyloid β. Ma et al. (Ma et al., Nature. 372, 92-94, 1994) have reported that ACT can enhance the aggregation of amyloid β 1-42, and these authors speculate that the enhanced aggregation contributes to its neurotoxicity.

Amyloid β Cellular Responses and *In Vivo* Pathology. Beyond the plaques and tangles that are the hallmarks of Alzheimer's disease, it is clear that a range of cellular responses has been induced, both in neurons and in accompanying glial cells. At a biochemical level, hyperphosphorylation of the tau protein is evident, resulting from perturbation of the kinase/phosphatase balance. At a transcriptional level, a variety of genes are activated to produce a spectrum of proteins not normally present or only present at lower levels in the brain. There also is significant evidence that inflammatory processes have been activated. In particular, tau phosphorylation has been documented to be induced by aggregated amyloid β 1-42 in differentiated SH-SY5Y cells (Lambert et al., J. Neurosci. Res., 39, 377-384, 1994), and this result has been confirmed in a more recent report by Busciglio et al. (Neuron, 14, 879-88, 1995), in which amyloid β activated tau phosphorylation in cultured primary rat hippocampal neurons.

Fibrillar Amyloid β and Neurodegeneration in Alzheimer's Disease. The mechanism by which amyloid β 1-42 causes Alzheimer's disease has not been elucidated, but the literature contains more than 200 studies of amyloid β neurotoxicity, many of which have been reviewed recently (e.g., Yankner et al., Neuron, 16, 921-32, 1996; Iversen et al., Biochemical Journal, 311, 1-16, 1995). The consensus view is that in order for amyloid β to be toxic, it must assemble into fibrillar structures (Pike et al., J. Neurosci., 13, 1676-87, 1993). Solutions containing only monomeric amyloid β have repeatedly been demonstrated to have no deleterious effect on neurons in culture. Furthermore, studies have correlated the formation of amyloid β-sheer containing fibrils and the timing and extent of toxicity using techniques such as circular dichroism and electron microscopy (Simmons et al., Molecular Pharmacology, 45, 373-9, 1994). One study concluded explicitly that amyloid β must exist in fibrillar form in order for it to be toxic (Lorenzo et al., Proc. Natl. Acad. Sci. USA, 91, 12243-12247, 1994). Despite this consensus regarding amyloid β structure and activity, there continues to be a problem of reproducibility of published experimental work involving amyloid toxicity (Brining, Neurobiology of Aging, 18, 581-589, 1997), and widespread variability of activity obtained with different batches of amyloid, or even the same batch of amyloid handled in slightly different ways, in spite of identical chemical composition (May et al., Neurobiology of Aging, 13, 1676-87, 1993). This has raised questions regarding the precise structures of amyloid β that are responsible for its activity.

The present invention seeks to overcome the problems in the prior art. Accordingly, it is an object of the present invention to provide a new composition of matter, amyloid β peptide assembled into soluble globular non-fibrillar oligomeric structures (ADDLs), that unexpectedly are neurotoxic. These and other objects and advantages of the present invention, as well as additional inventive features, will be apparent from the following description.

### BRIEF DESCRIPTION OF THE FIGURES

**Figure 1** is a computer-generated image of a densitometer-scanned silver-stained polyacrylamide gel which shows the ADDLs electrophoresing with a primary band corresponding to about 30 kD, a less abundant band corresponding to about 17 kD, and no evidence of fibrils or aggregates.
**Figure 2** is a computer-generated image of a densitometer-scanned Coomassie-stained SDS-polyacrylamide gel which shows ADDLs electrophoresing with a primary band (upper doublet) corresponding to a size of about 17 to about 22 kD, and with another band (lower dark band) indicating abundant 4 kD monomer present, presumably a breakdown product. *Lanes:* first, molecular size markers; second ADDL preparation; third, heavier loading of ADDL preparation.
**Figure 3** is a representative computer-generated image of AFM analysis of ADDL-containing "fraction 3" (fractionated on a Superdex 75 gel filtration column).
**Figure 4** is a computer-generated image of a densitometer-scanned Coomassie-stained SDS-polyacrylamide gradient gel of ADDLs prepared by coincubation with clusterin *(lane A)* or cold F12 media *(lane B),* and of ADDLs prepared by coincubation with clusterin and which passed through a Centricon 10 kD cut-off membrane *(lane C*) or were retained by a Centricon 10 kD cut-off membrane *(lane D):* **MW,** molecular size markers.
**Figure 5** is a graph of ADDL concentration measured as amyloid β 1-42 concentration (nM) vs. % dead cells for brain slices from mice treated with the ADDL preparations.
**Figure 6** is a bar chart showing % MTT reduction for control PC 12 cells not exposed to ADDLs ("Cont."), PC 12 cells exposed to clusterin alone ("Apo J"), PC 12 cells exposed to monomeric Aβ ("Aβ"), PC12 cells exposed to amyloid β coaggregated with clusterin and aged one day ("Aβ:Apo J").
**Figure 7** is a FACScan showing fluorescence intensity (0-170) versus events (0-300) for B103 cells not exposed to ADDLs (unshaded peak) and B103 cells bound to fluorescent labeled ADDLs (shaded peak).
**Figure 8** is a FACScan showing fluorescence intensity (0-200) versus events (0-300) for hippocampal cells not exposed to ADDLs (unshaded peak, "-ADDLs") and hippocampal cells bound to fluorescent labeled ADDLs (shaded peak, "+ADDL").
**Figure 9** is a bar chart of percent maximum ADDL binding or ADDL-evoked death for B 103 cells that either have been not exposed ("-") or coexposed ("+") to the peptides released by trypsinization of B 103 cells.
**Figure 10** is a graph of relative ADDL concentration vs. % dead cells for brain slices from mice treated with the ADDL preparations. To determine relative concentration, an initial concentration of 10 µM Aβ protein was employed to form ADDLs at the highest data point (point "16"), this was subsequently diluted to ½ (point "8"), ¼ (point "4"), and the like.
**Figure 11** is a bar chart showing optical density obtained in the ADDL binding ELISA assay wherein B103 cells were coincubated with ADDLs and 6E10 antibody ("cells, ADDL, 6E 10" bar), B103 cells were coincubated with ADDLs and ("cells, ADDL" bar), B103 cells were coincubated with 6E10 antibody ("cells, 6E10" bar), B103 cells were incubated alone ("cells" bar), 6E10 antibody was incubated alone ("6E10" bar), or the optical density of diluent was read ("blank" bar).
**Figure 12** is a bar chart of % dead cells in either *fyn* +/+ (wild type, "Fyn +"; crosshatched bars) or *fyn* -/- (knockout, "Fyn -"; solid bars) mice either not treated ("Medium") or contacted with ADDLs ("ADDLs").
**Figure 13** is a graph of Aβ concentration (µM) versus activated glia (number) obtained upon incubation of astrocytes with ADDLs (filled triangles) or Aβ 17-42 (filled squares).
**Figure 14** is a graph of time (minutes) versus % baseline cell body spike amplitude for control mice not treated with ADDLs (filled triangles) or mice treated with ADDLs (filled squares).
**Figure 15** is a graph of time (minutes) versus mean spike amplitude for control rat hippocampal slices not exposed to ADDLs (filled triangles) versus rat hippocampal slices exposed to ADDLs (filled squares).
**Figure 16** is a computer-generated image of a densitometer-scanned 16.5% tris-tricine SDS-polyacrylamide gel (Biorad) which shows a range of oligomeric, soluble ADDLs (labeled "ADDLs"), and amyloid β dimer (labeled "Dimer"), and monomer (labeled "Monomer"). *Lanes:* first, silver stained Mark XII molecular weight standards (Novex, San Diego, California); second, silver stained ADDLs; third, Western blot of second lane using the monoclonal antibody 26D6 (Sibia Neurosciences, San Diego, California).
**Figure 17** is a computer-generated image of an AFM analysis of ADDLs. The top view subtracted image shows a high magnification view (2.0 µm x 2.0 µm) of aggregated amyloid β molecules that have been spotted on freshly cleaved mica.

### SUMMARY OF THE INVENTION

The invention encompasses a composition of matter, termed amyloid beta-derived dementing ligands or amyloid beta-derived diffusible ligands (ADDLs) for use in the treatment of diseases, disorders, or conditions due to the effects of the ADDLs. ADDLs consist of 13-24 myloid β peptides assembled into soluble non-fibrillar oligomeric structures that are capable of activating specific cellular processes.

### DETAILED DESCRIPIPTION OF THE INVENTION

It has been discovered that in neurotoxic samples of amyloid β not only do fibrillar structures exist, but also, unexpectedly, some globular protein structures exist that appear to be responsible for the neurotoxicity. Using the methods herein, samples that contain predominantly these soluble globular protein assemblies and no fibrillar structures have been generated as described herein. In heterogeneous samples prepared by various methods, the removal of the larger, fibrillar forms of amyloid β by centrifugation does not remove these soluble globular assemblies of amyloid β in the supernatant fractions. These supernatant fractions exhibit significantly higher neurotoxicity than unfractionated amyloid β samples aggregated under literature conditions. These unexpected neurotoxic Soluble globular forms are referred to herein as amyloid β-derived dementing ligands, amyloid β-derived diffusible ligands (ADDLs), amyloid β soluble non-fibrillar amyloid β oligomeric structures, or simply oligomeric structures. Samples of amyloid β that had been "aged" under standard literature conditions (e.g., Pike et al., J. Neurosci., 13, 1676-1687, 1993) for more than three weeks lose their neurotoxicity, even though these samples contain predominantly fibrillar structures with few or no ADDLs. This discovery that the globular ADDLs are neurotoxic is particularly surprising since current thinking holds that it is fibril structures that constitute the toxic form of amyloid β (Lorenzo et al., Proc. Natl. Acad. Sci. USA, 91, 12243-12247, 1994; Howlett et al., Neurodegen, 4, 23-32, 1995).

The ADDLs can be formed in vitro. When a solution (e.g., a DMSO solution) containing monomeric amyloid β 1-42 (or other appropriate amyloid β, as further described herein) is diluted into cold tissue culture media (e.g., F12 cell culture media), then allowed to incubate at about 4°C for from about 2 to about 48 hours and centrifuged for about 10 minutes at about 14,000g at a temperature of 4°C, the supernatant fraction contains small, soluble oligomeric globules that are highly neurotoxic, e.g., in neuronal cell and brain slice cultures. The ADDLs also can be formed by coincubation of amyloid β with certain appropriate agents, e.g., clusterin (a senile plaque protein that also is known as ApoJ), as well as by other methods, as described herein.

Thus, in particular, the present invention provides an isolated soluble non-fibrillar amyloid β oligomeric structure for use in the treatment or diagnosis of Alzheimer's disease, Down's syndrome, mild cognitive impairment and senile demention and contains 13-24 amyloid B proteins. The oligomeric structure so isolated does not contain an exogenous added crosslinking agent. The oligomeric structure desirably is stable in the absence of any crosslinker.

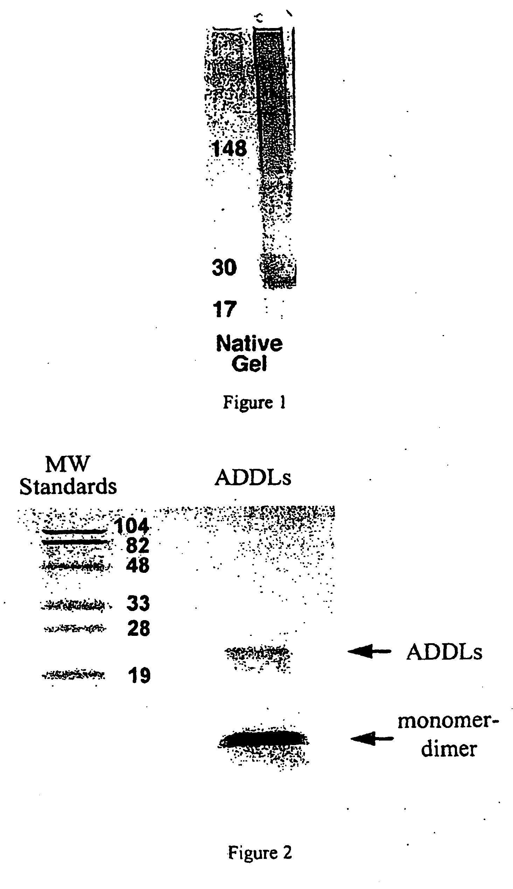

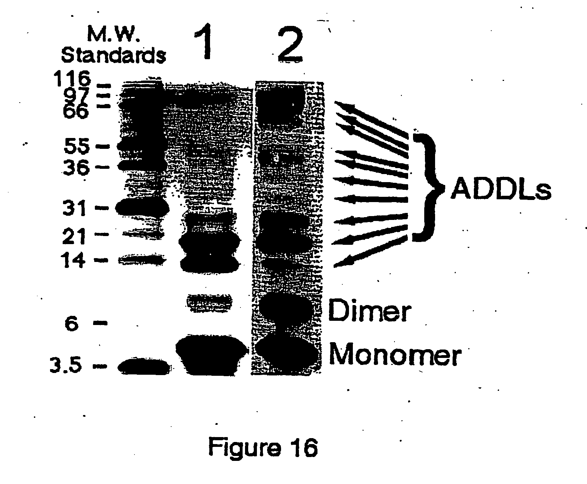

Atomic force microscope analysis (AFM) can be carried out as is known in the art and described herein, for instance, using a Digital Instruments Atomic force microscope as described in Example 3. AFM of such a supernatant fraction (i.e., a supernatant fraction in which fibrillar structures have been removed) reveals a number of different size globules (i.e., or different size oligomeric structures) present in the fraction. These globules fall within the range of from about 4.7 to about 11.0 nm, with the major fraction falling within a size range of from about 4.7 nm to about 6.2 nm. There appear to be distinct species of globules failing within this size range and which correspond to specific size oligomeric species such as those indicated by analysis on certain gel electrophoresis systems, as shown in **Figures 2** and 16. Slight variation in height surface results from how the particular species are seated on the mica surface at the time of AFM analysis. Despite this slight variation however, there appear to be several predominant sizes of globules in the 4.7-6.2 size range, i.e., from about 4.9 nm to about 5.4 nm, and from about 5.7 nm to about 6.2 nm, that constitute about 50% of the oligomeric structures in a typical sample. There also appears to be a distinct size species of globule having dimensions of from about 5,3 nm to about 5.7 nm. Larger globules from about 6.5 nm to about 11.0 nm appear less frequently, but may possess neurotoxic properties similar to the more prevalent, smaller species. It appears that the globules of dimensions of from about 4.7 nm to about 6.2 nm on AFM comprise the pentamer and hexamer form of oligomeric amyloid P (Aβ) protein. The AFM size globules of from about 4.2 nm to about 4.7 nm appear to correspond to the Aβ tetramer. The size globules of from about 3.4 nm to about 4.0 nm to appear to correspond to trimer. The large globules appear to correspond to oligomeric species ranging in size from about 13 amyloid monomes to about 24 amyloid monomers. The size globules of from about 2.8 nm to about 3.4 nm correspond to dimer (Roher et al., J. Biol. Chem., 271, 20631-20635, 1996). The Aβ monomer AFM size ranges from about 0.8 nm to about 1.8 - 2.0 nm. Monomeric and dimeric amyloid β are not neurotoxic in neuronal cell cultures or in the organotypic brain slice cultures.

Thus, the present invention provides an isolated soluble non-fibrillar amyloid β oligomeric structure (i.e., an ADDL) for the uses specified hereinabove that comprises at from 13 to 24 amyloid β protein monomers, especially from 13 to 20 amyloid β protein monomers, particularly from 13 to 16 amyloid β protein monomers, As previously described, large globules (less predominant species) appear to correspond to oligomeric species ranging in size from 13 amyloid β monomers to 24 amyloid β monomers. Accordingly, the isolated soluble non-fibrillar amyloid β oligomeric structure and in the invention comprises 16-mer, 20-mer or 24-mer aggregates of amyloid β proteins.

The oligomeric structure used in the invention optimally exhibits neurotoxic activity.

The higher order structure of the soluble non-fibrillar amyloid β protein oligomer structure (i.e., the aggregation of monomers to form the oligomeric structure) desirably can be obtained not only from amyloid β 1-42, but also from any amyloid β protein capable of stably forming the soluble non-fibrillar amyloid β oligomeric structure. In particular, amyloid β 1-43 also can be employed. Amyloid β 1-42 with biocytin at position 1 also can be employed. Amyloid β (e.g., β 1-42 or β 1-43) with a cysteine at the N-terminus also can be employed. Similarly, Aβ truncated at the amino terminus (e.g., particularly missing one or more up to the entirety of the sequence of amino acid residues 1 through 8 of Aβ 1-42 or Aβ 1-43), or Aβ (e.g., Aβ 1-42 or 1-43) having one or two extra amino acid residues at the carboxyl terminus can be employed. By contrast, amyloid β 1-40 can transiently form ADDL-like structures which can be toxic, but these structures are not stable and cannot be isolated as aqueous solutions, likely due to the shortened nature of the protein, which limits its ability to form such higher order assemblies in a stable fashion.

Desirably, the isolated soluble non-fibrillar amyloid β **o**ligomeric structure used in the invention comprises globules of dimensions of from about 4.7 nm to about 11.0 nm, particularly from about 4.7 nm to about 6.2 nm as measured by atomic force microscopy. Also, preferably the isolated soluble non-fibrillar amyloid β oligomeric structure comprises globules of dimensions of from about 4.9 nm to about 5.4 nm, or from about 5.7 nm to about 6.2 nm, or from about 6.5 nm to about 11.0 nm, as measured by atomic force microscopy. In particular, preferably the isolated soluble non-fibrillar amyloid β oligomeric structure used in the invention is such that wherein from about 30% to about 85%, even more preferably from about 40% to about 75% of the assembly comprises two predominant sizes of globules, namely, of dimensions of from about 4.9 nm to about 5.4 nm, and from about 5.7 nm to about 6.2 nm, as measured by atomic force microscopy. However, it also is desirable that the oligomeric structure comprises AFM size globules of about 5.3 to about 5.7 nm. It is also desirable that the oligomeric structure may comprise AFM size globules of about 6.5 run to about 11.0 nm.

By non-denaturing gel electrophoresis, the bands corresponding to ADDLs run at about from 26 kD to about 28 kD, and with a separate broad band representing sizes of from about 36 kD to about 108 kD. Under denaturing conditions (e.g., on a 15% SDS-polyacrylamide gel), the ADDLs comprise a band that runs at from about 22 kD to about 24 kD, and may further comprise a band that runs at about 18 to about 19 kD. Accordingly, the invention preferably provides an isolated soluble non-fibrillar amyloid β oligomeric structure (i.e., ADDL) that has a molecular weight of from about 26 kD to about 28 kD as determined by non-denaturing gel electrophoresis. The invention also preferably provides an isolated soluble non-fibrillar amyloid β oligomeric structure (i.e., ADDL) that runs as a band corresponding to a molecular weight of from about 22 kD to about 24 kD as determined by electrophoresis on a 15% SDS-polyacrylamide gel. The invention further preferably provides an isolated soluble non-fibrillar amyloid β oligomeric structure (i.e., ADDL) that runs as a band corresponding to a molecular weight of from about 18 kD to about 19 kD as determined by electrophoresis on a 15% SDS-polyacrylamide gel.

Also, using a 16.5% tris-tricine SDS-polyacrylamide gel system, additional ADDL bands can be visualized. The increased resolution obtained with this gel system confirms the ability to obtain according to the invention an isolated oligomeric structure having a molecular weight ranging from about 13 kD to about 116 kD, as determined by electrophoresis on a 16.5% tris-tricine SDS-polyacrylamide gel. The ADDL bands appear to correspond to distinct size species. In particular, use of this gel system allows visualization of bands corresponding to trimer with a size of about 13 to about 14 kD, tetramer trimer with a size of about 17 to about 19 kD, pentamer with a size of about 22 kD to about 23 kD, hexamer with a size of about 26 to about 28 kD, heptamer with a size from about 32 kD to 33 kD, and octamer with a size from about 36 kD to about 38 kD, as well as larger soluble oligomers ranging in size from about 12 monomers to about 24 monomers. Thus, the invention desirably provides an isolated oligomeric structure, wherein the oligomeric structure has, as determined by electrophoresis on a 16.5% tris-tricine SDS-polyacrylamide gel, a molecular weight selected from the group consisting of from about 13 kD to about 14 kD, from about 17 kD to about 19 kD, from about 22 kD to about 23 kD, from about 26 kD to about 28 kD, from about 32 kD to about 33 kD, and from about 36 kD to about 38 kD.

Disclosed herein is a method for preparing the isolated soluble non-fibrillar amyloid β oligomeric structure. This method optionally comprises the steps of:
(a) obtaining a solution of monomeric amyloid β protein;
(b) diluting the protein solution into an appropriate media;
(c) incubating the media resulting from step (b) at about 4°C;
(d) centrifuging the media at about 14,000 g at about 4°C; and
(e) recovering the supernatant resulting from the centrifugation as containing the amyloid β oligomeric structure. In step (c) of this method, the solution desirably is incubated for from about 2 hours to about 48 hours, especially from about 12 hours to about 48 hours, and most preferably from about 24 hours to about 48 hours. In step (d) of this method, the centrifugation preferably is carried out for from about 5 minutes to about 1 hour, especially for from about 5 minutes to about 30 minutes, and optimally for about 10 minutes. Generally, however, this is just a precautionary measure to remove any nascent fibrillar or protofibrillar structures and may not be necessary, particularly where long-term stability of the ADDL preparation is not an issue.

The Aβ protein is diluted in step (b) desirably to a final concentration ranging from about 5 nM to about 500 µM, particularly from about 5µM to about 300 µM, especially at about 100 µM. The "appropriate media" into which the Aβ protein solution is diluted in step (b) preferably is any media that will support, if not facilitate, ADDL formation. In particular, F12 media (which is commercially available as well as easily formulated in the laboratory) is preferred for use in this method

Similarly, "substitute F12 media" also desirably can be employed. Substitute F 12 media differs from F12 media that is commercially available or which is formulated in the laboratory. Substitute F12 media preferably comprises the following components: N,N-dimethylglycine, D-glucose, calcium chloride, copper sulfate pentahydrate, iron(II) sulfate heptahydrate, potassium chloride, magnesium chloride, sodium chloride, sodium bicarbonate, disodium hydrogen phosphate, and zinc sulfate heptahydrate.

In particular, synthetic F12 media optionally comprises: N, N-dimethylglycine (from about 600 to about 850 mg/L), D-glucose (from about 1.0 to about 3.0 g/L), calcium chloride (from about 20 to about 40 mg/L), copper sulfate pentahydrate (from about 15 to about 40 mg/L), iron (II) sulfate heptahydrate (from about 0.4 to about 1.2 mg/L), potassium chloride (from about 160 to about 280 mg(L), magnesium chloride (from about 40 to about 75 mg/L), sodium chloride (from about 6.0 to about 9.0 g/L), sodium bicarbonate (from about 0.75 to about 1.4 g/L), disodium hydrogen phosphate (from about 120 to about 160 mg/L), and zinc sulfate heptahydrate (from about 0.7 to about 1.1 mg/L). Optimally, synthetic F12 media comprises: N, N-dimethylglycine (about 766 mg/L), D-glucose (about 1.802 g/L), calcium chloride (about 33 mg/L), copper sulfate pentahydrate (about 25 mg/L), iron(II) sulfate heptahydrate (about 0.8 mg/L), potassium chloride (about 223 mg/L), magnesium chloride (about 57 mg/L), sodium chloride (about 7.6 g/L), sodium bicarbonate (about 1.18 g/L), disodium hydrogen phosphate (about 142 mg/L), and zinc sulfate heptahydrate (about 0.9 mg/L). Further, the pH of the substitute F12 media preferably is adjusted, for instance, using 0.1 M sodium hydroxide, desirably to a pH of from about 7.0 to about 8.5, and preferably a pH of about 8.0.

The foregoing method further desirably can be carried out by forming the slowly-sedimenting oligomeric structure in the presence of an appropriate agent, such as clusterin. This is done, for instance, by adding clusterin in step (c), and, as set out in the Examples which follow.

Moreover, also disclosed herein and as described in the Examples, is a method for preparing a soluble non-fibrillar amyloid β oligomeric structure used in the invention, wherein the method comprises:
(a) obtaining a solution of monomeric amyloid β protein, the amyloid β protein being capable of forming the oligomeric structure;
(b) dissolving the amyloid β monomer in hexafluoroisoproanol;
(c) removing hexafluoroisoproanol by speed vacuum evaporation to obtain solid peptide;
(d) dissolving the solid peptide in DMSO to form a DMSO stock solution;
(e) diluting the stock solution into an appropriate media;
(f) vortexing; and
(g) incubating at about 4°C for about 24 hours.

If the ADDLs are prepared by the incorporation of 10% biotinylated amyloid β 1-42 (or other appropriate biotinylated amyloid β protein), they can be utilized in a receptor binding assay using neural cells and carried out, for instance, on a fluorescence activated cell sorting (FACS) instrument, with labeling by a fluorescent avidin conjugate. Alternately, instead of incorporating biotin in the amyloid β protein, another reagent capable of binding the ADDL to form a fluorescently labeled molecule, and which may already be part of a fluorescent-labeled conjugate, can be employed. For instance, the soluble non-fibrillar amyloid β oligomeric structure can be formed such that the amyloid protein includes another binding moiety, with "binding moiety" as used herein encompassing a molecule (such as avidin, streptavidin, polylysine, and the like) that can be employed for binding to a reagent to form a fluorescently-labeled compound or conjugate. The "fluorescent reagent" to which the oligomeric structure binds need not itself fluoresce directly, but instead may merely be capable of fluorescence through binding to another agent For example, the fluorescent reagent which binds the oligomeric structure can comprise a β amyloid specific antibody (e.g., 6E10), with fluorescence generated by use of a fluorescent secondary antibody.

Along with other experiments, FACSscan analysis of the rat CNS B103 cells was done without and with ADDL incubation. Results of these and further studies confirm that binding to the cell surface is saturable, and brief treatment with trypsin selectively removes a subset of cell surface proteins and eliminates binding of ADDLs. Proteins that are cleavable by brief treatment with trypsin from the surface of B103 cells also prevent ADDL binding to B103 cells or cultured primary rat hippocampal neurons. These results all support that the ADDLs act through a particular cell surface receptor, and that early events mediated by the ADDLs (i.e., events prior to cell killing) can be advantageously controlled (e.g., for treatment or research) by compounds that block formation and activity (e.g., including receptor binding) of the ADDLs.

Thus, disclosed herein is a method for identifying compounds that modulate (i.e., either facilitate or block) activity (e.g., activity such as receptor binding) of the ADDL. This method preferably comprises:
(a) contacting separate cultures of neuronal cells with the oligomeric structure of the invention either in the presence or absence of contacting with the test compound;
(b) adding a reagent that binds to the oligomeric structure, the reagent being fluorescent;
(c) analyzing the separate cell cultures by fluorescence-activated cell sorting; and
(d) comparing the fluorescence of the cultures, with compounds that block activity (i.e., binding to a cell surface protein) of the oligomeric structure being identified as resulting in a reduced fluorescence of the culture, and compounds that facilitate binding to a cell surface protein (i.e., a receptor) being identified as resulting in an increased fluorescence of the culture, as compared to the corresponding culture contacted with the oligomeric structure in the absence of the test compound. Alternately, instead of adding a fluorescent reagent that in and of itself is able to bind the protein complex, the method desirably is carried out wherein the oligomeric structure is formed from amyloid β 1-42 protein (or another amyloid β) prepared such that it comprises a binding moiety capable of binding the fluorescent reagent.

Similarly, the method can be employed for identifying compounds that modulate (i.e., either facilitate or block) formation or activity (e.g., binding to a cell surface protein, such as a receptor) of the oligomeric structure comprising:
(a) preparing separate samples of amyloid β that either have or have not been mixed with the test compound;
(b) forming the oligomeric structure in the separate samples;
(c) contacting separate cultures of neuronal cells with the separate samples;
(d) adding a reagent that binds to the oligomeric structure, the reagent being fluorescent;
(e) analyzing the separate cell cultures by fluorescence-activated cell sorting; and
(f) comparing the fluorescence of the cultures, with compounds that block formation or binding to a cell surface protein of the oligomeric structure being identified as resulting in a reduced fluorescence of the culture, and compounds that facilitate formation or binding to a cell surface protein of the oligomeric structure being identified as resulting in an increased fluorescence of the culture, as compared to the corresponding culture contacted with the oligomeric structure in the absence of the test compound. Further, instead of adding a fluorescent reagent that in and of itself is able to bind the protein complex, the method can be carried out wherein the oligomeric structure is formed from amyloid β protein prepared such that it comprises a binding moiety capable of binding the fluorescent reagent.

The fluorescence of the cultures further optionally is compared with the fluorescence of cultures that have been treated in the same fashion except that instead of adding or not adding test compound prior to formation of the oligomeric structure, the test compound either is or is not added after formation of the oligomeric structure. In this situation, compounds that block formation of the oligomeric structure are identified as resulting in a reduced fluorescence of the culture, and compounds that facilitate formation of the oligomeric structure are identified, as resulting in an increased fluorescence of the culture, as compared to the corresponding culture contacted with the oligomeric structure in the absence of the test compound, only when the compound is added prior to oligomeric structure.

By contrast, compounds that block binding to a cell surface protein (e.g., a receptor) of the oligomeric structure are identified as resulting in a reduced fluorescence of the culture, and compounds that facilitate binding to a cell surface protein of the oligomeric structure are identified as resulting in an increased fluorescence of the culture, as compared to the corresponding culture contacted with the oligomeric structure in the absence of the test compound, when the compound is *added either prior to or after* oligomeric structure.

In a similar fashion, a cell-based assay, particularly a cell-based enzyme-linked immunosorbent assay (ELISA) can be employed to assess ADDL binding activity. In particular, the method can be employed to detect binding of the oligomeric structure to a cell surface protein. This method preferably comprises:
(a) forming an oligomeric structure from amyloid β protein;
(b) contacting a culture of neuronal cells with the oligomeric structure;
(c) adding an antibody (e.g., 6E10) that binds said oligomeric structure, said antibody including a conjugating moiety (e.g., biotin, or other appropriate agent);
(d) washing away unbound antibody;
(e) linking an enzyme (e.g., horseradish peroxidase) to said antibody bound to said oligomeric structure by means of said conjugating moiety;
(f) adding a colorless substrate (e.g., ABTS) that is cleaved by said enzyme to yield a color change; and
(g) determining said color change (e.g., spectrophotometrically) or the rate of the color change as a measure of binding to a cell surface protein (e.g., a receptor) of said oligomeric structure. As earlier described, the antibody can be any antibody capable of detecting ADDLs (e.g., an antibody directed to an exposed site on amyloid β), and the antibody conjugating moiety can be any agent capable of linking a means of detection (e.g., an enzyme). The enzyme can be any moiety (e.g., perhaps even other than a protein) that provides a means of detecting (e.g., color change due to cleavage of a substrate), and further, can be bound (e.g., covalent or noncovalent) to the antibody bound to the oligomeric structure by means of another moiety (e.g., a secondary antibody). Also, preferably the cells are adhered to a solid substrate (e.g., tissue culture plastic) prior to the conduct of the assay. It goes without saying that desirably step (b) should be carried out as described herein such that ADDLs are able to bind to cells. Similarly, preferably step (c) should be carried out for a sufficient length of time (e.g., from about 10 minutes to about 2 hours, desirably for about 30 minutes) and under appropriate conditions (e.g., at about room temperature, preferably with gentle agitation) to allow antibody to bind to ADDLs. Further, appropriate blocking steps can be carried out such as are known to those skilled in the art using appropriate blocking reagents to reduce any nonspecific binding of the antibody. The artisan is familiar with ELISAs and can employ modifications to the assay such as are known in the art.

The assay desirably also can be carried out so as to identify compounds that modulate (i.e., either facilitate or block) formation or binding to a cell surface protein of the oligomeric structure. In this method, as in the prior-described assays for test compounds, the test compound is either added to the ADDL preparation, prior to the contacting of the cells with the ADDLs. This assay thus can be employed to detect compounds that modulate formation of the oligomeric structure (e.g., as previously described). Moreover, the test compound can be added to the ADDL preparation prior to contacting the cells (but after ADDL formation), or to the cells prior to contact with ADDLs. This method (e.g., as previously described) can be employed to detect compounds that modulate ADDL binding to the cell surface. Also, a test compound can be added to the mixture of cells plus ADDLs. This method (e.g., as previously described) can be employed to detect compounds that impact on ADDL-mediated events occurring downstream of ADDL binding to a cell surface protein (e.g., to an ADDL receptor). The specificity of the compounds for acting on an ADDL-mediated downstream effect can be confirmed, for instance, by simply adding the test compound in the absence of any coincubation with ADDLs. Of course, further appropriate controls (e.g., as set forth in the following Examples and as known to those skilled in the art) should be included with all assays.

Similarly, using the methods described herein (e.g., in the Examples), compounds that block formation of the oligomeric structure used in the invention, may be identified by a method that desirably comprises: _{.}
(a) preparing separate samples of amyloid β protein that either have or have not been mixed with the test compound;
(b) forming the oligomeric structure in the separate samples
(c) assessing whether any protein assemblies have formed in the separate samples using a method selected from the group consisting of electrophoresis, immunorecognition, and atomic force microscopy; and
(d) comparing the formation of the protein assemblies in the separate samples, which compounds that block formation of the oligomeric structure being identified as resulting in decreased formation of the oligomeric structure in the sample as compared with a sample in which the oligomeric structure is formed in the absence of the test compound.

This information on compounds the modulate (i.e., facilitate or block) formation and/or activity including binding to a cell surface protein of the oligomeric structure can be employed in the research and treatment of ADDL-mediated diseases, conditions, or disorders. The methods described herein can be employed to investigate the activity and neurotoxicity of the ADDLs themselves. For instance, when 20 nL of the ADDL preparation was injected into the hippocampal region of an adult mouse 60-70 minutes prior to the conduct of a long-term potentiation (LTP) experiment (e.g. Namgung et al., Brain Research, 689, 85-92, 1995), the stimulation phase of the experiment occurred in a manner identical with saline controls injections, but the consolidation phase showed a significant, continuing decline in synaptic activity as measured by cell body spike amplitude, over the subsequent 2 hours, compared with control animals, in which synaptic activity remained at a level comparable to that exhibited during the stimulation phase. Analysis of brain slices after the experiment indicated that no cell death had occurred. These results, as well as other described in the following Examples, confirm that ADDL treatment compromised the LTP response. This indicates that ADDLs contribute to the compromised learning and memory observed in Alzheimer's disease by interference with neuronal signaling processes, rather than by the induction of nerve cell death.

Additional information on the effects of ADDLs (either in the presence or absence of test compounds that potentially modulate ADDL formation and/or activity) can be obtained using the further assays described herein For instance, a method for assaying the effects of ADDLs preferably comprises:
(a) administering the oligomeric structure to the hippocampus of an animal;
(b) applying an electrical stimulus; and
(c) measuring the cell body spike amplitude over time to determine the long-term potentiation response. The method optionally is carried out wherein the long-term potentiation response of the animal is compared to the long-term potentiation response of another animal treated in the same fashion except having saline administered instead of oligomeric structure prior to application of the electrical stimulus. This method further can be employed to identify compounds that modulate (i.e., increase or decrease) the effects of the ADDLs, for instance, by comparing the LTP response in animals administered ADDLs either alone, or, in conjunction with test compounds.

Along these lines, disclosed herein is a method for identifying compounds that modulate the effects of the ADDL oligomeric structure. The method preferably comprises:
(a) administering either saline or a test compound to the hippocampus of an animal;
(b) applying an electrical stimulus;
(c) measuring the cell body spike amplitude over time to determine the long-term potentiation response; and
(d) comparing the long-term potentiation response of animals having saline administered to the long-term potentiation response of animals having test compound administered. The method further optionally comprises administering oligomeric structure to the hippocampus either before, along with, or after administering the saline or test compound.

Similarly, compounds that modulate (i.e., either increase or decrease) the neurotoxicity of the ADDL protein assembly, may be identified by a method wich comprises:
(a) contacting separate cultures of neuronal cells with the oligomeric structure either in the presence or absence of contacting with the test compound;
(b) measuring the proportion of viable cells in each culture; and
(c) comparing the proportion of viable cells in each culture. Compounds that block the neurotoxicity of the oligomeric structure are identified, for example, as resulting in an increased proportion of viable cells in the culture as compared to the corresponding culture contacted with the oligomeric structure in the absence of the test compound. Compounds that increase the neurotoxicity of the oligomeric structure are identified, for example, as resulting in a reduced portion of viable cells in the culture as compared to the corresponding culture contacted with the oligomeric structure in the presence of the test compound.

The methods disclosed herein also can be employed in detecting in test materials the ADDLs (e.g., as part of research, diagnosis, and/or therapy). For instance, ADDLs bring about a rapid morphological change in serum-starved B 103 celts, and they also activate Fyn kinase activity in these cells within 30 minutes of ADDL treatment (data not shown). ADDLs also induce rapid complex formation between Fyn and focal adhesion kinase (FAK; Zhang et al, Neurosci. Letters, 211, 1-4, 1996), and translocating of several phosphorylated proteins and Fyn-Fak complex to a Triton-insoluble fraction (Berg et al., J. Neurosci. Res., 50, 979-989, 1997). This suggests that Fyn and other activated signaling pathways are involved in the neurodegenerative process induced by ADDLs. This has been confirmed by experiment in brain slice cultures from genetically altered mice that lack a functional *fyn* gene, where addition of ADDLs resulted in no increased neurotoxicity compared to vehicle controls.

Therefore, compounds that block one or more of Fyn's function, or Fyn relocalization namely by impacting on ADDLs, may be important neuroprotective drugs for Alzheimer's disease. Similarly, when ADDLs are added to cultures of primary astrocytes, the astrocytes become activated and the mRNA for several proteins, including IL-1, inducible nitric oxide synthase, Apo B, Apo J and α1-antichymotrypsin become elevated. These phenomena desirably are employed in a method for detecting in a test material the ADDL protein assembly. Such methods optionally comprise:
(a) contacting the test material with an antibody (e.g., the 6E10 antibody or another antibody); and
(b) detecting binding to the oligomeric structure of the antibody.

Similarly, the method desirably can be employed wherein
(a) the test material is contacted with serum-starved neuroblastoma cells (e.g., B103 neuroblastoma cells); and
(b) morphological changes in the cells are measured by comparing the morphology of the cells against neuroblastoma cells that have not been contacted with the test material.

The method also preferably can be employed wherein:
(a) the test material is contacted with brain slice cultures; and
(b) brain cell death is measured as compared against brain slice cultures that have not been contacted with the test material. The method further desirably can be conducted wherein:
   (a) the test material is contacted with neuroblastoma cells (e.g., B103 neuroblastoma cells); and
   (b) increases in *fyn* kinase activity are measured by comparing *fyn* kinase activity in the cells against *fyn* kinase activity in neuroblastoma cells that have not been contacted with said test material. In particular, Fyn kinase activity can be compared making use of a commercially available kit (e.g., Kit #QIA-28 from Oncogene Research Products, Cambridge, MA) or using an assay analogous to that described in Borowski et al., J. Biochem. (Tokyo), 115, 825-829, 1994.

In yet another preferred embodiment of the method of detecting ADDLs in test material, the method desirably comprises:
(a) contacting the test material with cultures of primary astrocytes; and
(b) determining activation of the astrocytes as compared to cultures of primary astrocytes that have not been contacted with the test material. In a variation of this method, the method optionally comprises:
   (a) contacting the test material with cultures of primary astrocytes; and
   (b) measuring in the astrocytes increases in the mRNA for proteins selected from the group consisting of interleukin-1, inducible nitric oxide synthase, Apo E, Apo J, and α1-antichymotrypsin by comparing the mRNA levels in the astrocytes against the corresponding mRNA levels in cultures of primary astrocytes that have not been contacted with the test material. There are, of course, other methods of assay, and further variations of those described above that would be apparent to one skilled in the art, particularly in view of the specification disclosure herein.

Thus, clearly, the ADDLs used in the present invention have utility in *vitro.* Such ADDLs can be used *inter alia* as a research tool in the study of ADDL binding and interaction within cells and in a method of assaying ADDL activity. Similarly, ADDLs, and studies of ADDL formation, activity and modulation can be employed in vivo.

In particular, the compounds identified using the methods described herein can be used to treat any one of a number of diseases, disorders or conditions that result in deficits in cognition or learning (i.e., due to a failure of memory), and/or deficits in memory itself. Such treatment or prevention can be effected by administering compounds that prevent formation and/or activity of the ADDLs, or that modulate (i.e., increase or decrease the activity of, desirably as a consequence of impacting ADDLs) the cell agents with which the ADDLs interact (e.g., so-called "downstream" events). Such compounds having ability to impact ADDLs are referred to herein as "ADDL-modulating compounds,". ADDL-modulating compounds not only can act in a negative fashion, but also, in some cases preferably are employed to increase the formation and/or activity of the ADDLs.

Desirably, when employed *in vivo,* the method can be employed for protecting an animal against decreases in cognition, learning or memory due to the effects of the ADDL protein assembly. This method comprises administering a compound that blocks the formation or activity of the ADDLs. Similarly, to the extent that deficits in cognition, learning and/or memory accrue due to ADDL formation and/or activity, such deficits can be reversed or restored once the activity (and/or formation) of ADDLs is blocked. The invention thus provides a composition for use in a method for reversing (or restoring) in an animal decreases in cognition, learning or memory due to the effects of an oligomeric structure used in the invention. This method preferably comprises blocking the formation or activity of the ADDLs. The invention thus also provides a composition for use in a method for reversing in a nerve cell decreases in long-term potentiation due to the effects of a soluble non-fibrillar amyloid β oligomeric structure used in the invention (as well as protecting a nerve cell against decrease in long-term potentiation due to to the effects of a soluble non-fibrillar amyloid β oligomeric structure), the method comprising contacting the cell with a compound that blocks the formation or activity of the oligomeric structure.

In particular, this method desirably can be applied in the treatment or prevention of a disease, disorder, or condition that manifests as a deficit in cognition, learning and/or memory and which is due to ADDL formation or activity, especially a disease, disorder, or condition selected from the group consisting of Alzheimer's disease, adult Down's syndrome (i.e., over the age of 40 years), and senile dementia.

Also, this method desirably can be applied in the treatment or prevention of early deleterious effects on cellular activity, cognition, learning, and memory that may be apparent prior to the development of the disease, disorder, or condition itself, and which deleterious effects may contribute to the development of, or ultimately constitute the disease, disorder, or condition itself. In particular, the method preferably can be applied in the treatment or prevention of the early malfunction of nerve cells or other brain cells that can result as a consequence of ADDL formation or activity. Similarly, the method preferably can be applied in the treatment or prevention of focal memory deficits (FMD) such as have been described in the literature (e.g., Linn et al., Arch. Neurol., 52, 485-490, 1995), in the events such FMD are due to ADDL formation or activity. The method further desirably can be employed in the treatment or prevention of ADDL-induced aberrant neuronal signaling, impairment of higher order writing skills (e.g., Snowdon et al., JAMA, 275, 528-532, 1996) or other higher order cognitive function, decreases in (or absence of) long-term potentiation, that follows as a consequence of ADDL formation or activity.

As disclosed herein, "ADDL-induced aberrant neuronal signaling" can be measured by a variety of means. For instance, for normal neuronal signaling (as well as observation of a long-term potentiation response), it appears that among other things, Fyn kinase must be activated, Fyn kinase must phosphorylate the NMDA channel (Miyakawa et al., Science. 278, 698-701, 1997; Grant, J Physiol Paris, 90, 337-338, 1996), and Fyn must be present in the appropriate cellular location (which can be impeded by Fyn-FAK complex formation, for instance, as occurs in certain cytoskeletal reorganizations induced by ADDL). Based on this, ADDL-induced aberrant neuronal signaling (which is a signaling malfunction that is induced by aberrant activation of cellular pathways by ADDLs) and knowledge thereof can be employed in the methods of the invention, such as would be obvious to one skilled in the art. For instance, ADDL-induced aberrant cell signaling can be assessed (e.g., as a consequence of contacting nerve cells with ADDLs, which may further be conducted in the presence or absence of compounds being tested for ADDL-modulating activity) using any of these measures, or such as would be apparent to one skilled in the art, e.g., Fyn kinase activation (or alteration thereof, Fyn-FAK complex formation (or alteration thereof), cytoskeletal reorganization (or alteration thereof, Fyn kinase subcellular localization (or alteration thereof). Fyn kinase phosphorylation of the NMDA channel (or alteration thereof).

Also, the ADDLs themselves may be applied in treatment. It has been discovered that these novel assemblies described herein have numerous unexpected effects on cells that conceivably can be applied for therapy. For instance, ADDLs activate endothelial cells, which endothelial cells are known, among other things to interact with vascular cells. Along these lines, ADDLs could be employed, for instance, in wound healing. Also, by way of example, Botulinum Toxin Type A (BoTox) is a neuromuscular junction blocking agent produced by the bacterium *Clostridium botulinum* that acts by blocking the release of the neurotransmitter acetylcholine. Botox has proven beneficial in the treatment of disabling muscle spasms, including dystonia. ADDLs themselves theoretically could be applied to either command neural cell function or, to selectively destroy targeted neural cells (e.g., in cases of cancer, for instance of the central nervous system, particularly brain). ADDLs appear further advantageous in this regard given that they have very early effects on cells, and given that their effect on cells (apart from their cell killing effect) appears to be reversible.

As discussed above, the ADDL-modulating compounds of the present invention, compounds known to impact incorporation of amyloid β into higher molecular weight structures, as well as ADDLs themselves, can be employed to contact cells either *in vitro* or *in vivo.* According to the invention, a cell can be any cell, and, preferably, is a eukaryotic cell. A eukaryotic cell is a cell typically that possesses at some stage of its life a nucleus surrounded by a nuclear membrane. Preferably the eukaryotic cell is of a multicellular species (e.g., as opposed to a unicellular yeast cell), and, even more preferably, is a mammalian (optionally human) cell. However, the method also can be effectively carried out using a wide variety of different cell types such as avian cells, and mammalian cells including but not limited to rodent, primate (such as chimpanzee, monkey, ape, gorilla, orangutan, or gibbon), feline, canine, ungulate (such as ruminant or swine), as well as, in particular, human cells. Preferred cell types are cells formed in the brain, including neural cells and glial cells. An especially preferred cell type according to the invention is a neural cell (either normal or aberrant, e.g., transformed or cancerous). When employed in tissue culture, desirably the neural cell is a neuroblastoma cell.

A cell can be present as a single entity, or can be part of a larger collection of cells. Such a "larger collection of cells" can comprise, for instance, a cell culture (either mixed or pure), a tissue (e.g., neural or other tissue), an organ (e.g., brain or other organs), an organ system (e.g., nervous system or other organ system), or an organism (e.g., mammal, or the like). Preferably, the organs/tissues/cells of interest in the context of the invention are of the central nervous system (e.g., are neural cells).

Also, according to the invention "contacting" comprises any means by which these agents physically touch a cell. The method is not dependent on any particular means of introduction and is not to be so construed. Means of introduction are well known to those skilled in the art, and also are exemplified herein. Accordingly, introduction can be effected, for instance, either *in vitro* (e.g., in an ex vivo type method of therapy or in tissue culture studies) or *in vivo*. Other methods also are available and are known to those skilled in the art.

Such "contacting" can be done by any means known to those skilled in the art, and described herein, by which the apparent touching or mutual tangency of the ADDLs and ADDL-modulating compounds and the cell can be effected. For instance, contacting can be done by mixing these elements in a small volume of the same solution. Optionally, the elements further can be covalently joined, e.g., by chemical means known to those skilled in the art, or other means, or preferably can be linked by means of noncovalent interactions (e.g., ionic bonds, hydrogen bonds, Van der Waals forces, and/or nonpolar interactions). In comparison, the cell to be affected and the ADDL or ADDL-modulating compound need not necessarily be brought into contact in a small volume, as, for instance, in cases where the ADDL or ADDL-modulating compound is administered to a host, and the complex travels by the bloodstream or other body fluid such as cerebrospinal fluid to the cell with which it binds. The contacting of the cell with a ADDL or ADDL-modulating compound sometimes is done either before, along with, or after another compound of interest is administered. Desirably this contacting is done such that there is at least some amount of time wherein the coadministered agents concurrently exert their effects on a cell or on the ADDL.

One skilled in the art will appreciate that suitable methods of administering an agent (e.g., an ADDL or ADDL-modulating compound) of the present invention to an animal for purposes of therapy and/or diagnosis, research or study are available, and, although more than one route can be used for administration, a particular route can provide a more immediate and more effective reaction than another route. Pharmaceutically acceptable excipients also are well-known to those who are skilled in the art, and are readily available. The choice of excipient will be determined in part by the particular method used to administer the agent. Accordingly, there is a wide variety of suitable formulations for use in the context of the present invention. The following methods and excipients are merely exemplary and are in no way limiting.

Formulations suitable for oral administration can consist of (a) liquid solutions, such as an effective amount of the compound dissolved in diluents, such as water, saline, or orange juice; (b) capsules, sachets or tablets, each containing a predetermined amount of the active ingredient, as solids or granules; (c) suspensions in an appropriate liquid; and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, emulsions, gels, and the like containing, in addition to the active ingredient, such excipients as are known in the art.

An agent of the present invention, alone or in combination with other suitable ingredients, can be made into aerosol formulations to be administered via inhalation. These aerosol formulations can be placed into pressurized acceptable propellants, such as dichlorodifluoromethane, propane, nitrogen, and the like. They also can be formulated as pharmaceuticals for non-pressured preparations such as in a nebulizer or an atomizer.

Formulations suitable for parenteral administration are preferred according to the invention and include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

The dose administered to an animal, particularly a human, in the context of the present invention will vary with the agent of interest, the composition employed, the method of administration, and the particular site and organism being treated. However, preferably a dose corresponding to an effective amount of an agent (e.g., an ADDL or ADDL-modulating compound according to the invention) is employed. An "effective amount" is one that is sufficient to produce the desired effect in a host, which can be monitored using several end-points known to those skilled in the art. Some examples of desired effects include, but are not limited to, an effect on learning, memory, LTP response, neurotoxicity, ADDL formation, ADDL cell surface protein (e.g., receptor) binding, antibody binding, cell morphological changes, Fyn kinase activity, astrocyte activation, and changes in mRNA levels for proteins such as interleukin-1, inducible nitric oxide synthase, ApoE, ApoJ, and α1-antichymotrypsin. These methods described are by no means all-inclusive, and further methods to suit the specific application will be apparent to the ordinary skilled artisan.

Moreover, with particular applications (e.g., either *in vitro* or *in vivo*) the actual dose and schedule of administration of ADDLs or ADDL-modulating compounds can vary depending on whether the composition is administered in combination with other pharmaceutical compositions, or depending on interindividual differences in pharmacokinetics, drug disposition, and metabolism. Similarly, amounts can vary in *in vitro* applications depending on the particular cell type utilized or the means or solution by which the ADDL or ADDL-modulating compound is transferred to culture. One skilled in the art easily can make any necessary adjustments in accordance with the requirements of the particular situation.

With use of certain compounds, it may be desirable or even necessary to introduce the compounds (i.e., agents) as pharmaceutical compositions directly or indirectly into the brain. Direct techniques include, but are not limited to, the placement of a drug delivery catheter into the ventricular system of the host, thereby bypassing the blood-brain barrier. Indirect techniques include, but are not limited to, the formulation of the compositions to convert hydrophilic drugs into lipid-soluble drugs using techniques known in the art (e.g., by blocking the hydroxyl, carboxyl, and primary amine groups present on the drug) which render the drug able to cross the blood-brain barrier. Furthermore, the delivery of hydrophilic drugs can be improved, for instance, by intra-arterial infusion of hypertonic solutions (or other solutions) which transiently open the blood brain barrier.

### Examples

The foregoing descriptions (as well as those which follow) are exemplary only. Other applications of the method and constituents of the present invention will be apparent to one skilled in the art. Thus, the following examples further illustrate the present invention but, of course, should not be construed as in any way limiting the scope.

### Example 1: Preparation of Amyloid β Oligomers

According to the invention, ADDLs were prepared by dissolving 1 mg of solid amyloid β1-42 (e.g., synthesized as described in Lambert et al., J. Neurosci. Res., 39, 377-395, 1994) in 44 µL of anhydrous DMSO. This 5 mM solution then was diluted into cold (4°C) F12 media (Gibco BRL, Life Technologies) to a total volume of 2.20 mL (50-fold dilution), and vortexed for about 30 seconds. The mixture was allowed to incubate at from about 0°C to about 8°C for about 24 hours, followed by centrifugation at 14,000g for about 10 minutes at about 4°C. The supernatant was diluted by factors of 1:10 to 1:10,000 into the particular defined medium, prior to incubation with brain slice cultures, cell cultures or binding protein preparations. In general, however, ADDLs were formed at a concentration of Aβ protein of 100 µM. Typically, the highest concentration used for experiments is 10 µM and, in some cases, ADDLs (measured as initial Aβ concentration) were diluted (e.g., in cell culture media) to 1 nM. For analysis by atomic force microscopy (AFM), a 20 µL aliquot of the 1:100 dilution was applied to the surface of a freshly cleaved mica disk and analyzed. Other manipulations were as described as follows, or as is apparent.

Alternately, ADDL formation was carried out as described above, with the exception that the F12 media was replaced by a buffer (i.e., "substitute F12 media") containing the following components: N, N-dimethylglycine (766 mg/L), D-glucose (1.802 g/L), calcium chloride (33 mg/L), copper sulfate pentahydrate (25 mg/L), iron(II) sulfate heptahydrate (0.8 mg/L), potassium chloride (223 mg/L), magnesium chloride (57 mg/L), sodium chloride (7.6 g/L), sodium bicarbonate (1.18 g/L),disodium hydrogen phosphate (142 mg/L), and zinc sulfate heptahydrate (0.9 mg/L). The pH of the buffer was adjusted to 8.0 using 0.1 M sodium hydroxide.

### Example 2: Crosslinking of Amyloid β Oligomers (comparative)

Glutaraldehyde has been successfully used in a variety of biochemical systems. Glutaraldehyde tends to crosslink proteins that are directly in contact, as opposed to nonspecific reaction with high concentrations of monomeric protein. In this example, glutaraldehyde-conunanded crosslinking of amyloid β was investigated.

Oligomer preparation was carried out as described in example 1, with use of substitute F12 media. The supernatant that was obtained following centrifugation (and in some cases, fractionation) was treated with 0.22 mL of a 25% aqueous solution of glutaraldehyde (Aldrich), followed by 0.67 mL of 0.175 M sodium borohydride in 0.1 M NaOH (according to the method of Levine, Neurobiology of Aging, 1995). The mixture was stirred at 4°C for 15 minutes and was quenched by addition of 1.67 mL of 20% aqueous sucrose. The mixture was concentrated 5 fold on a SpeedVac and dialyzed to remove components smaller than 1 kD. The material was analyzed by SDS PAGE. Gel filtration chromatography was carried according to the following: Superose 75PC 3.2/3.0 column (Pharmacia) was equilibrated with filtered and degassed 0.15% ammonium hydrogen carbonate buffer (pH=7.8) at a flow rate of 0.02 mL/min over the course of 18 h at room temperature. The flow rate was changed to 0.04 mL/min and 20 mL of solvent was eluted. 50 microliters of reaction solution was loaded on to the column and the flow rate was resumed at 0.04 mL/min. Compound elution was monitored via UV detection at 220 nm, and 0.5-1.0 mL fractions were collected during the course of the chromatography. Fraction No. 3, corresponding to the third peak of UV absorbance was isolated and demonstrated by AFM to contain globules 4.9 +/- 0.8 nm (by width analysis). This fraction was highly neurotoxic when contacted with brain slice neurons, as described in the examples which follow.

### Example 3: Size Characterization of ADDLs

This example sets forth the size characterization of ADDLs formed as in Example 1, and using a variety of methods (e.g., native gel electophoresis, SDS-polyacrylamide gel electrophoresis, AFM, field flow fractionation, and immunorecognition).

AFM was carried out essentially as described previously (e.g., Stine et al., J. Protein Chem., 15, 193-203, 1996). Namely, images were obtained using a Digital Instruments (Santa Barbara, CA) Nanoscope IIIa Multimode Atomic force microscope using a J-scanner with xy range of 150µ. Tapping Mode was employed for all images using etched silicon TESP Nanoprobes (Digital Instruments). AFM data is analyzed using the Nanoscope IIIa software and the IGOR Pro^{™} waveform analysis software. For AFM analysis, 4 µ scans (i.e., assessment of a 4 µm x 4 µm square) were conducted. Dimensions reported herein were obtained by section analysis, and where width analysis was employed, it is specified as being a value obtained by width analysis. Section and width analysis are in separate analysis modules in the Nanoscope IIIa software. Generally, for ADDL analysis, there is a systematic deviation between the sizes obtained by section analysis and those obtained by width analysis. Namely, for a 4 µ scan, section analysis yields heights that are usually about 0.5 nm taller, thus resulting in a deviation of about 0.5 nm in the values obtained for the sizes of the globules.

Analysis by gel electrophoresis was carried out on 15% polyacrylamide gels and visualized by Coomassie blue staining. ADDLs were resolved on 4-20% tris-glycine gels under non-denaturing conditions (Novex). Electrophoresis was performed at 20 mA for approximately 1.5 hours. Proteins were resolved with SDS-PAGE as described in Zhang et al., J. Biol. Chem., 269, 25247-25250, 1994. Protein was then visualized using silver stain (e.g. as described in Sherchenko et al., Anal. Chem., 68, 850-858, 1996). Gel proteins from both native and SDS gels were transferred to nitrocellulose membranes according to Zhang et al. (J. Biol. Chem., 269, 25247-50, 1994). Immunoblots were performed with biotinylated 6E10 antibody (Senetak, Inc., St. Louis, Missouri) at 1:5000 and visualized using ECL (Amersham). Typically, gels were scanned using a densitometer. This allowed provision of the computer-generated images of the gels (e.g., versus photographs of the gels themselves).

Size characterization of ADDLs by AFM section analysis (e.g., as described in Stine et al., J. Protein Chem., 15, 193-203, 1996) or width analysis (Nanoscope III software) indicated that the predominant species were globules of about 4.7 nm to about 6.2 nm along the z-axis. Comparison with small globular proteins (Aβ 1-40 monomer, aprotinin, bFGF, carbonic anhydrase) suggested that ADDLs had mass between 17-42 kD. What appear to be distinct species can be recognized. These appear to correspond to globules of dimensions of from about 4.9 nm to about 5.4 nm, from about 5.4 nm to about 5.7 nm, and from about 5.7 nm to about 6.2 nm. The globules of dimensions of about 4.9-5.4 nm and 5.7-6.2 nm appear to comprise about 50% of globules.

In harmony with the AFM analysis, SDS-PAGE immunoblots of ADDLs identified Aβ oligomers of about 17 kD to about 22 kD, with abundant 4 kD monomer present, presumably a breakdown product. Consistent with this interpretation, non-denaturing polyacrylamide gels of ADDLs show scant monomer, with a primary band near 30 kD, a less abundant band at ∼17 kD, and no evidence of fibrils or aggregates. Computer-generated images of a silver stained native gel and a Coomassie stained SDS-polyacrylamide gel are set out in **Figure 1** and **Figure 2****,** respectively. The correspondence between the SDS and non-denaturing gels confirms that the small oligomeric size of ADDLs was not due to detergent action. Oligomers seen in ADDL preparations were smaller than clusterin (Mr 80 kD, 40 kD in denatured gels), as expected from use of low clusterin concentrations (1/40 relative to Aβ, which precluded association of Aβ as 1:1 Aβ-clusterin complexes).

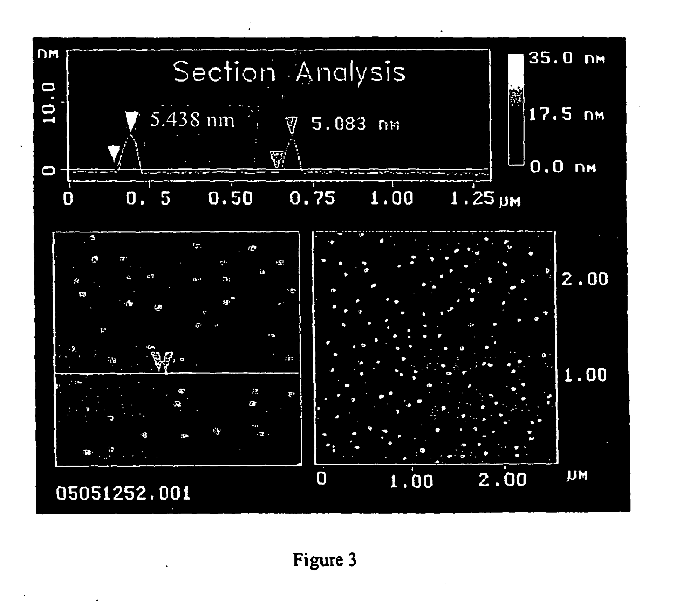

An ADDL preparation according to the invention was fractionated on a Superdex 75 column (Pharmacia, Superose 75PC 3.2/3.0 column). The fraction comprising the ADDLs was the third fraction of UV absorbance eluting from the column and was analyzed by AFM and SDS-polyacryalamide gel electrophoresis. A representative AFM analysis of fraction 3 is depicted in **Figure 3****.** Fractionation resulted in greater homogeneity for the ADDLs, with the majority of the globules having dimensions of from about 4.9 nm to about 5.4 nm. SDS-polyacrylamide gel electrophoresis of the fraction demonstrated a heavy lower band corresponding to the monomer/dimer form of Aβ. As also observed for the non-fractionated preparation of ADDLs, this appears to be a breakdown product of the ADDLs. Heavier loading of the fraction revealed a larger-size broad band (perhaps a doublet). This further confirms the stability of the non-fibrillar oligomeric Aβ structures to SDS.

### Example 4: Clusterin Treatment of Amyloid β

Although it has been proposed that fibrillar structures represent the toxic form of Aβ (Lorenzo et al., Proc. Natl. Acad Sci. USA, 91, 12243-12247, 1994; Howlett et al., Neurodegen. 4, 23-32, 1995), novel neurotoxins that do not behave as sedimentable fibrils will form when Aβ 1-42 is incubated with low doses of clusterin, which also is known as "Apo J" (Oda et al., Exper. Neurol., 136, 22-31, 1995; Oda et al., Biochem. Biophys. Res. Common., 204, 1131-1136, 1994). To test if these slowly sedimenting toxins might still contain small or nascent fibrils, clusterin-treated Aβ preparations were examined by atomic force microscopy.

Clusterin treatment was carried out as described in Oda et al. (Exper. Neurol., 136, 22-31, 1995) basically by adding clusterin in the incubation described in Example 1. Alternatively, the starting Aβ 1-42 could be dissolved in 0.1 N HCl, rather than DMSO, and this starting Aβ 1-42 could even have fibrillar structures at the outset. However, incubation with clusterin for 24 hours at room temperature of 37 °C resulted in preparations that were predominantly free of fibrils, consistent with their slow sedimentation. This was confirmed by experiments showing that fibril formation decreases as the amount of clusterin added increases.

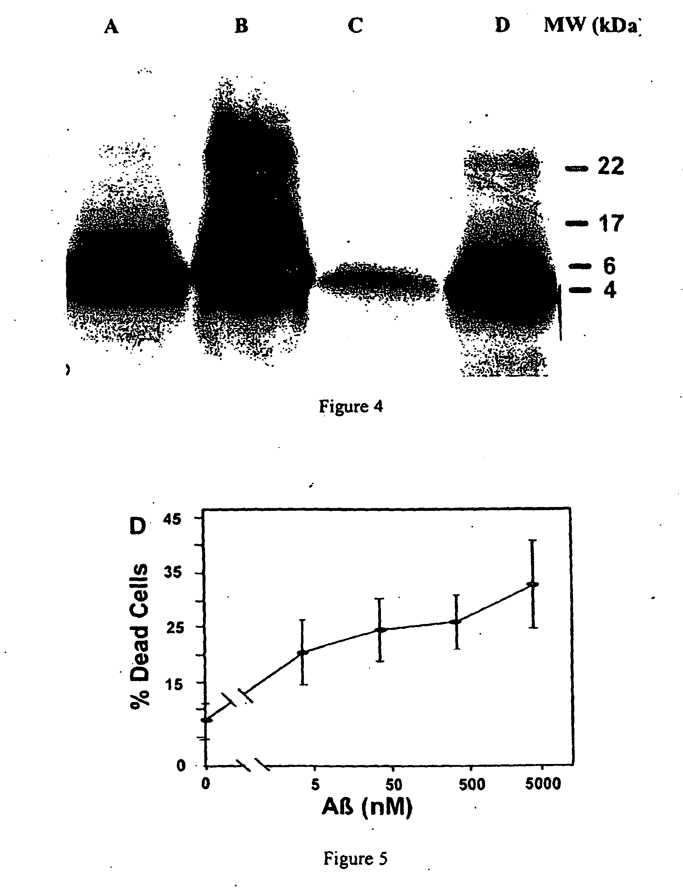

The preparations resulting from clusterin treatment exclusively comprised small globular structures approximately 5-6 nm in size as determined by AFM analysis of ADDLs fractionated on a Superdex 75 gel column. Equivalent results were obtained by conventional electron microscopy. In contrast, Aβ 1-42 that had self-associated under standard conditions (Snyder et al., Biophys. J., 67, 1216-28, 1994) in the absence of clusterin showed primarily large, non-diffusible fibrillar species. Moreover, the resultant ADDL preparations were passed through a Centricon 10 kD cut-off membrane and analyzed on as SDS-polyacrylamide gradient gel. As can be seen in **Figure 4****,** only the monomer passes through the Centricon 10 filter, whereas ADDLs are retained by the filter. Monomer found after the separation could only be formed from the larger molecular weight species retained by the filter.

These results confirm that toxic ADDL preparations comprise small fibril-free oligomers of Aβ1-42, and that ADDLs can be obtained by appropriate clusterin treatment of amyloid β.

### Example 5: Physiologic Formation of ADDLs

The toxic moieties in Example 4 could comprise rare structures that contain oligomeric Aβ and clusterin. Whereas Oda et al. (Exper. Neurol., 136,22-31, 1995) reported that clusterin was found to increase the toxicity of Aβ 1-42 solutions, others have found that clusterin at stoichiometric levels protects against Aβ 1-40 toxicity (Boggs et al., J. Neurochem., 67, 1324-1327, 1997). Accordingly, ADDL formation in the absence of clusterin further was characterized in this Example.

When monomeric Aβ 1-42 solutions were maintained at low temperature in an appropriate media, formation of sedimentable Aβ fibrils was almost completely blocked. Aβ, however, did self-associate in these low-temperature solutions, forming ADDLs essentially indistinguishable from those chaperoned by clusterin. Finally, ADDLs also formed when monomeric Aβ solutions were incubated at 37 degrees in brain slice culture medium but at very low concentration (50 nM), indicating a potential to form physiologically. All ADDL preparations were relatively stable and showed no conversion to fibrils during the 24 hour tissue culture experiments.

These results confirm that ADDLs form and are stable under physiological conditions and suggest that they similarly can form and are stable *in vivo.*

### Example 6: ADDLS are Diffusible, Extremely Potent CNS Neurotoxins

Whether ADDLs were induced by clusterin, low temperature, or low Aβ concentration, the stable oligomers that formed were potent neurotoxins. Toxicity was examined in organotypic mouse brain slice cultures, which provided a physiologically relevant model for mature CNS. Brain tissue was supported at the atmosphere-medium interface by a filter in order to maintain high viability in controls.

For these experiments, brain slices were obtained from strains B6 129 F2 and JR 2385 (Jackson Laboratories) and cultured as previously described (Stoppini et al., J. Neurosci. Meth., 37, 173-182, 1991), with modifications. Namely, an adult mouse was sacrificed by carbon dioxide inhalation, followed by rapid decapitation. The head was emersed in cold, sterile dissection buffer (94 mL Gey's balanced salt solution, pH 7.2, supplemented with 2 mL 0.5M MgCl₂, 2 ml 25% glucose, and 2 mL 1.0 M Hepes), after which the brain was removed and placed on a sterile Sylgard-coated plate. The cerebellum was removed and a mid-line cut was made to separate the cerebral hemispheres. Each hemisphere was sliced separately. The hemisphere was placed with the mid-line cut down and a 30 degree slice from the dorsal side was made to orient the hemisphere. The hemisphere was glued cut side down on the plastic stage of a Campden tissue chopper (previously wiped with ethanol) and emersed in ice cold sterile buffer. Slices of 200 µm thickness were made from a lateral to medial direction, collecting those in which the hippocampus was visible.

Each slice was transferred with the top end of a sterile pipette to a small petri dish containing Dulbecco's Modified Eagle Medium (DMEM) containing 10% fetal calf serum, 2% S/P/F (streptomycin, penicillin, and fungizone; Life Technologies (Gibco, BRL), Gaithersburg, MD), observed with a microscope to verify the presence of the hippocampus, and placed on a Millicell-CM insert (Millipore) in a deep well tissue culture dish (Falcon, 6-well dish). Each well contained 1.0 mL of growth medium, and usually two slices were on each insert. Slices were placed in a incubator (6% CO₂, 100% humidity) overnight. Growth medium was removed and wells were washed with 1.0 mL warm Hanks BSS (Gibco, BRL, Life Technologies). Defined medium (DMEM, N2 supplements, SPF, e.g., as described in Bottenstein et al., Proc. Natl. Acad. Sci., 76, 514-517, 1979) containing the amyloid β oligomers, with or without inhibitor compounds, was added to each well and the incubation was continued for 24 hours.

Cell death was measured using the **LIVE/DEAD**® assay kit (Molecular Probes, Eugene, OR). This a dual-label fluorescence assay in which live cells are detected by the presence of an esterase that cleaves calcein-AM to calcein, resulting in a green fluorescence. Dead cells take up ethidium homodimer, which intercalates with DNA and has a red fluorescence. The assay was carried out according to the manufacturer's directions at 2 µM ethidium homodimer and 4 µM calcein. Images were obtained within 30 minutes using a Nikon Diaphot microscope equipped with epifluorescence. The MetaMorph image analysis system (Universal Imaging Corporation, Philadelphia, PA) was used to quantify the number and/or area of cells showing green or red fluorescence.

For these experiments, ADDLs were present for 24 hours at a maximal 5 µM dose of total Aβ (i.e., total Aβ was never more than 5 µM in any ADDL experiment). Cell death, as shown by "false yellow staining", was almost completely confined to the stratum pyramidale (CA 3-4) and dentate gyrus (DG) suggesting strongly that principal neurons of the hippocampus (pyramidal and granule cells, respectively) are the targets of ADDL-induced toxicity. Furthermore, glia viability is unaffected by a 24 hour ADDL treatment of primary rat brain glia, as determined by trypan blue exclusion and MTT assay (Finch et al., unpublished). Dentate gyrus (DG) and CA3 regions were particularly sensitive and showed ADDL-evoked cell death in every culture obtained from animals aged P20 (weanlings) to P84 (young adult). Up to 40% of the cells in this region die following chronic exposure to ADDLs. The pattern of neuronal death was not identical to that observed for NMDA, which killed neurons in DG and CA1 but spared CA3.

Some cultures from hippocampal DG and CA3 regions of animals more than 20 days of age were treated with conventional preparations of fibrillar Aβ. Consistent with the non-diffusible nature of the fibrils, no cell death (yellow staining) was evident even at 20 µM. The staining pattern for live cells in this culture verified that the CA3/dentate gyrus region of the hippocampus was being examined. The extent of cell death observed after conventional Aβ treatment (i.e., fibrillar Aβ preparations) was indistinguishable from negative controls in which cultures were given medium, or medium with clusterin supplement. In typical controls, cell death was less than 5%. In fact, high viability in controls could be found even in cultures maintained several days beyond a typical experiment, which confirms that cell survival was not compromised by standard culture conditions.

A dose-response experiment was carried out to determine the potency of ADDLs in evoking cell death. Image analysis was used to quantify dead cell and live cell staining in fields containing the DG/CA3 areas. **Figure 5** illustrates the % dead cells verses ADDL concentration measured as initial amyloid β 1-42 concentration (nM). Because of the difficulties of quantifying brain slices, the results are not detailed enough to determine the EC50 with precision. However, as can be seen in **Figure 5****,** even after 1000-fold dilution ( ∼ 5 nM Aβ), ADDL-evoked cell death was more than 20%. Toxicity was observed even with 0.3 nM ADDLs. This contrasts with results obtained with conventionally aged Aβ, which is toxic to neurons in culture at about 20 to about 50 µM. These data show that ADDLs are effective at doses approximately 1,000-10,000-fold smaller than those used in fibrillar Aβ experiments.

These data from hippocampal slices thus confirm the ultratoxic nature of ADDLs. Furthermore, because ADDLs had to pass through the culture-support filter to cause cell death, the results validate that ADDLs are diffusible, consistent with their small oligomeric size. Also, the methods set forth herein can be employed as an assay for ADDL-mediated changes in cell viability. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 7: MTT Oxidative Stress Toxicity Assay - PC12 Cells

This example sets forth an assay that can be employed to detect an early toxicity change in response to amyloid β oligomers.

For these experiments, PC12 cells were passaged at 4 x 10⁴ cells/well on a 96-well culture plate and grown for 24 hours in DMEM + 10% fetal calf serum + 1% S/P/F (streptomycin, penicillin, and fungizone). Plates were treated with 200 µg/mL poly-1-lysine for 2 hours prior to cell plating to enhance cell adhesion. One set of six wells was left untreated and fed with fresh media, while another set of wells was treated with the vehicle control (PBS containing 10% 0.01 N HCl, aged o/n at RT). Positive controls were treated with triton (1%) and Na Azide (0.1%) in normal growth media. Amyloid β oligomers prepared as described in Example 1, or obtained upon coincubation with clusterin, with and without inhibitor compounds present, were added to the cells for 24 hours. After the 24 hour incubation, MTT (0.5 mg/mL) was added to the cells for 2.5 hours (11 µL of 5 mg/ml stock solubilized in PBS into 100 µL of media). Healthy cells reduce the MTT into a formazan blue colored product. After the incubation with MTT, the media was aspirated and 100 µL of 100% DMSO was added to lyse the cells and dissolve the blue crystals. The plate was incubated for 15 min at RT and read on a plate reader (ELISA) at 550 nm.

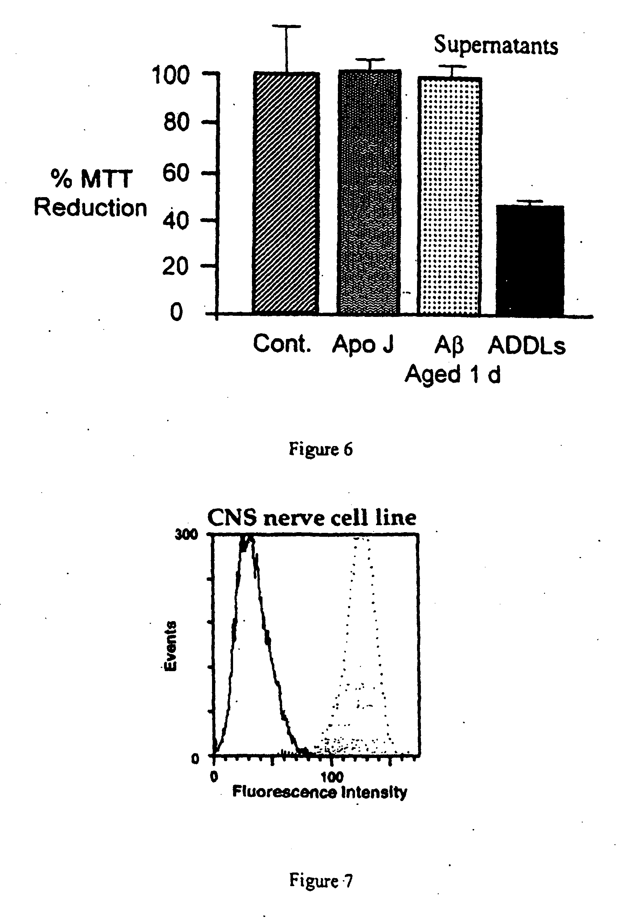

The results of one such experiment are depicted in **Figure 6****.** As can be seen from this figure, control cells not exposed to ADDLs ("Cont."), cells exposed to clusterin alone ("Apo J"), and cells exposed to monomeric Aβ ("Aβ") show no cell toxicity. By contrast, cells exposed to amyloid β coaggregated with clusterin and aged one day ("Aβ:Apo J") show a decrease in MTT reduction, evidencing an early toxicity change. The lattermost amyloid preparations were confirmed by AFM to lack amyloid fibrils.

Results of this experiment thus confirm that that ADDL preparations obtained from coaggregation of Aβ mediated by clusterin have enhanced toxicity. Moreover, the results confirm that the PC 12 oxidative stress response can be employed as an assay to detect early cell changes due to ADDLs. The assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 8: MTT Oxidative Stress Toxicity Assay- HN2 Cells

This example sets forth a further assay of ADDL-mediated cell changes. Namely, the MTT oxidative stress toxicity assay presented in the preceding example can be carried out with HN2 cells instead of PC 12 cells. Other appropriate cells similarly can be employed.

For this assay, HN2 cells were passaged at 4 x 10⁴ cells/well on a 96-well culture plate and grown for 24 hours in DMEM + 10% fetal calf serum + 1% S/P/F (streptomycin, penicillin, and fungizone). Plates were treated with 200 µg/mL poly 1-lysine for 2 hours prior to cell plating to enhance cell adhesion. The cells were differentiated for 24-48 hours with 5 µM retinoic acid and growth was further inhibited with 1% serum. One set of wells was left untreated and given fresh media. Another set of wells was treated with the vehicle control (0.2% DMSO). Positive controls were treated with triton (1%) and Na Azide (0.1%). Amyloid β oligomers prepared as described in example 1, with and without inhibitor compounds present, were added to the cells for 24 hours. After the 24 hour incubation, MTT (0.5 mg/mL) was added to the cells for 2.5 hours (11 µL of 5 mg/mL stock into 100 µL of media). After the incubation with MTT, the media was aspirated and 100 µL of 100% DMSO is added to lyse the cells and dissolve the blue crystals. The plate was incubated for 15 minutes at RT and read on a plate reader (ELISA) at 550 nm.

This assay similarly can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 9: Cell Morphology by Phase Microscopy

This example sets forth yet another assay of ADDL-mediated cell changes - assay of cell morphology by phase microscopy.

For this assay, cultures were grown to low density (50-60% confluence). To initiate the experiment, the cells were serum-starved in F12 media for 1 hour. Cells were then incubated for 3 hours with amyloid β oligomers prepared as described in example 1, with and without inhibitor compounds added to the cells, for 24 hours. After 3 hours, cells were examined for morphological differences or fixed for immunofluorescence labeling. Samples were examined using the MetaMorph Image Analysis system and an MRI video camera (Universal Imagine, Inc.).

Results of such assays are presented in the examples which follow. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 10: FACScan Assay for Binding of ADDLs to Cell Surfaces

Because cell surface receptors recently have been identified on glial cells for conventionally prepared Aβ (Yan et al., Nature. 382, 685-691, 1996; EI Khoury et al., Nature. 382, 716-719, 1996), and because neuronal death at low ADDL doses suggested possible involvement of signaling mechanisms, experiments were undertaken to determine if specific cell surface binding sites on neurons exist for ADDLs.

For flow cytometry, cells were dissociated with 0.1% trypsin and plated at least overnight onto tissue culture plastic at low density. Cells were removed with cold phosphate buffered saline (PBS) /0.5 mM EDTA, washed three times and resuspended in ice-cold PBS to a final concentration of 500,000 cells/mL. Cells were incubated in cold PBS with amyloid β oligomers prepared as described in Example 1, except that 10% of the amyloid β is an amyloid β 1-42 analog containing biocytin at position 1 replacing aspartate. Oligomers with and without inhibitor compounds present were added to the cells for 24 hours. The cells were washed twice in cold PBS to remove free, unbound amyloid β oligomers, resuspended in a 1:1,000 dilution of avidin conjugated to fluorescein, and incubated for one hour at 4°C with gentle agitation. Alternately, amyloid β-specific antibodies and fluorescent secondary antibody were employed instead of avidin, eliminating the need to incorporate 10% of the biotinylated amyloid β analog. Namely, biotinylated 6E10 monoclonal antibody (1µL Senetec, Inc., St. Louis, Missouri) was added to the cell suspension and incubated for 30 minutes. Bound antibody was detected after pelleting cells and resuspending in 500 µL PBS, using FITC-conjugated streptavidin (1:500, Jackson Laboratories) for 30 minutes.

Cells were analyzed by a Becton-Dickenson Fluorescence Activated Cell Scanner (FACScan). 10,000 or 20,000 events typically were collected for both forward scatter (size) and fluorescence intensity, and the data were analyzed by Consort 30 software (Becton-Dickinson). Binding was quantified by multiplying mean fluorescence by total number of events, and subtracting value for background cell fluorescence in the presence of 6E10 and FITC.

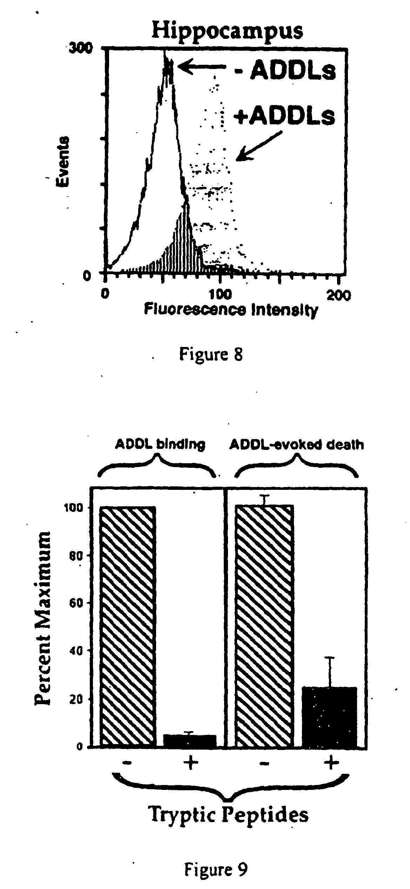

For these experiments, FACScan analysis was done to compare ADDL immunoreactivity in suspensions of log-phase yeast cells (a largely carbohydrate surface) and of the B103 CNS neuronal cell line (Schubert et al., Nature, 249, 224-227, 1974). For B103 cells, addition of ADDLs caused a major increase in cell associated fluorescence, as shown in **Figure 7****.** Trypsin treatment of the B103 cells for 1 minute eliminated ADDL binding. In contrast, control yeast cells (data not shown) demonstrated no ADDL binding, verifying the selectivity of ADDLs for proteins present on the cell surface. Suspensions of hippocampal cells (trypsinized tissue followed by a two-hour metabolic recovery) also bound ADDLs, but with a reduced number of binding events compared with the B103 cells, as evidenced by the reduced fluorescence intensity of the labelled peak. This appears in **Figure 8** as a leftward shifting of the labelled peak.

These results thus suggest that the ADDLs exert their effects by binding to a specific cell surface receptor. In particular, the trypsin sensitivity of B 103 cells showed that their ADDL binding sites were cell surface proteins and that binding was selective for a subset of particular domains within these proteins.

Moreover, the present assay can also be employed as an assay for ADDL-mediated cell binding. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 11: Inhibition of ADDL Formation by Gossypol

This example sets forth the manner in which ADDL formation can be inhibited using, for instance, gossypol.

For these experiments, ADDLs were prepared as described in Example 1. Gossypol (Aldrich) was added to a concentration of 100 µM during the incubation of the Aβ protein to form ADDLs. The resulting preparation was assessed for neurotoxicity using the **LIVE/DEAD**® assay kit as previously described. The amount of cell death that occurred after 24 hours of exposure to the gossypol/ADDL preparation was less than 5%. This is comparable to the level of toxicity obtained for a corresponding DMSO control preparation (i.e., 6%), or a gossypol control preparation that did not contain any ADDLs (i.e., 4%).

These results thus confirm that compounds such as gossypol can be employed to inhibit ADDL formation.

### Example 12: Inhibition of ADDL Binding by Tryptic Peptides

Because B 103 cell trypsinization was found to block subsequent ADDL binding, experiments were done as set forth in this example to test if tryptic fragments released from the cell surface retard ADDL binding activity.

Tryptic peptides were prepared using confluent B103 cells from four 100 mm dishes that were removed by trypsinization (0.025%, Life Technologies) for approximately 3 minutes. Trypsin-chymotrypsin inhibitor (Sigma, 0.5 mg/mL in Hank's Buffered Saline) was added, and cells were removed via centrifugation at 500 x g for 5 minutes. Supernatant (∼12 mL) was concentrated to approximately 1.0 mL using a Centricon 3 filter (Amicon), and was frozen after the protein concentration was determined. For blocking experiments, sterile concentrated tryptic peptides (0.25 mg/mL) were added to the organotypic brain slice or to the suspended B103 cells in the FACs assay at the same time as the ADDLs were added.

In FACScan assays, tryptic peptides released into the culture media (0.25 mg/mL) inhibited ADDL binding by > 90% as shown in **Figure 9****.** By comparison, control cells exposed to BSA, even at 100mg/mL, had no loss of binding. Tryptic peptides, if added after ADDLs were already attached to cells, did not significantly lower fluorescence intensities. This indicates that the peptides did not compromise the ability of the assay to quantify bound ADDLs. Besides blocking ADDL binding, the tryptic peptides also were antagonists of ADDL-evoked cell death. Namely, as shown in **Figure 9**, addition of tryptic peptides resulted in a 75% reduction in cell death, p < 0.002.

These data confirm that particular cell surface proteins mediate ADDL binding, and that solubilized tryptic peptides from the cell surface provide neuroprotective, ADDL-neutralizing activity. Moreover, the present assay can also be employed as an assay for agents that mediate ADDL cell binding or ADDL effects on cell activity. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone. Moreover, addition of the agents before or after binding of the ADDLs to the cell surface can be compared to identify agents that impact such binding, or that act after binding has occurred.

### Examples 13: Dose Response Curve for ADDL Cell Binding

This example sets forth dose response experiments done to determine whether ADDL binding to the cell surface is saturable. Such saturability would be expected if the ADDLs in fact interact with a particular cell surface receptor.

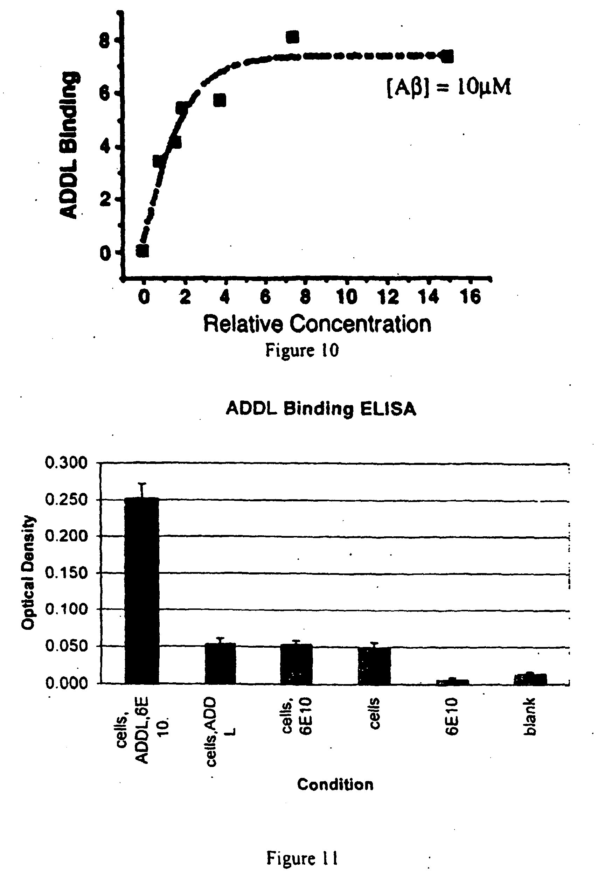

For these studies, B 103 cells were incubated with increasing amounts of ADDLs and ADDL binding was quantitated by FACscan analysis. Results are presented in **Figure 10****.** These results confirm that a distinct plateau is achieved for ADDL binding. Saturability of ADDL binding occurs at a relative Aβ 1-42 concentration (i.e., ADDL concentration relative to Aβ) of about 250 nm.

These results thus confirm that ADDL binding is saturable. Such saturability of ADDL binding, especially when considered with the results of the trypsin studies, validates that the ADDLs are acting through a particular cell surface receptor.

### Example 14: Cell-Based ELISA for ADDL Binding Activity

This example sets forth a cell-based assay, particularly a cell-based enzyme-linked immunosorbent assay (ELISA) that can be employed to assess ADDL binding activity.

For these studies, 48 hours prior to conduct of the experiment, 2.5 x 10⁴ B103 cells present as a suspension in 100 µL DMEM were placed in each assay well of a 96-well microtiter plate and kept in an incubator at 37°C. 24 hours prior to the conduct of the experiment, ADDLs were prepared according to the method described in Example 1 To begin the assay, each microtiter plate well containing cells was treated with 50µL of fixative (3.7% formalin in DMEM) for 10 minutes at room temperature. This fixative/DMEM solution was removed and a second treatment with 50 µL formalin (no DMEM) was carried out for 15 minutes at room temperature. The fixative was removed and each well was washed twice with 100 µL phosphate buffered saline (PBS). 200 µL of a blocking agent (1% BSA in PBS) was added to each well and incubated at room temperature for I hour. After 2 washes with 100 µL PBS, 50 µL of ADDLs (previously diluted 1:10 in PBS), were added to the appropriate wells, or PBS alone as a control, and the resulting wells were incubated at 37°C for 1 hour. 3 washes with 100 µL PBS were carried out, and 50 µL biotinylated 6E10 (Senetek) diluted 1:1000 in 1% BSA/PBS was added to the appropriate wells. In other wells, PBS was added as a control. After incubation for 1 hour at room temperature on a rotator, the wells were washed 3 times with 50 µL PBS, and 50 µL of the ABC reagent (Elite ABC kit, Vector Labs) was added and incubated for 30 minutes at room temperature on the rotator. After washing 4 times with 50 µL PBS, 50 µL of ABTS substrate solution was added to each well and the plate was incubated in the dark at room temperature. The plate was analyzed for increasing absorption at 405 nm. Only when ADDLs, cells, and 6E10 were present was there a significant signal, as illustrated in **Figure 11****.**

These results further confirm that a cell-based ELISA assay can be employed as an assay for ADDL-mediated cell binding. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 15: Fyn kinase knockout protects against ADDL neurotoxicity

To investigate further the potential involvement of signal transduction in ADDL toxicity, the experiments in this example compared the impact of ADDLs on brain slices from isogenic *fyn* -/- and *fyn* +/+ animals. Fyn belongs to the Src-family of protein tyrosine kinases, which are central to multiple cellular signals and responses (Clarke et al., Science, 268, 233-238). Fyn is of particular interest because it is upregulated in AD-afflicted neurons (Shirazi et al., Neuroreport, 4, 435-437, 1993). It also appears to be activated by conventional Aβ preparations (Zhang et al., Neurosci. Letts., 211, 187-190, 1996) which subsequently have been shown to contain ADDLs by AFM. Fyn knockout mice, moreover, have reduced apoptosis in the developing hippocampus (Grant et al., Science, 258, 1903-1910, 1992).

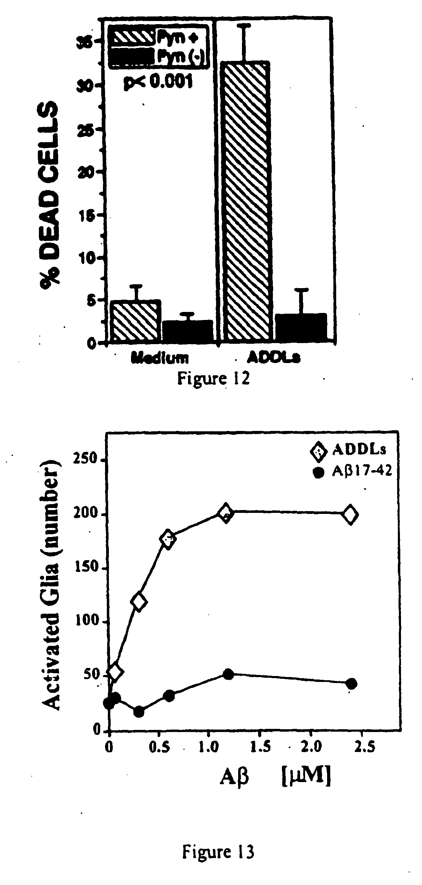

For these studies, Fyn knockout mice (Grant et al., Science, 258, 1903-1910, 1992) were treated as described in the preceding examples, by comparing images of brain slices of mice either treated or not treated with ADDLs for 24 hours to determine dead cells in the DG and CA3 area. The quantitative comparison (presented in **Figure 12**) was obtained with error bars representing means +/-SEM for 4-7 slices.

In contrast to cultures from wild-type animals, cultures from *fyn* -/- animals showed negligible ADDL-evoked cell death, as shown in **Figure 12****.** For ADDLs, the level of cell death in *fyn* +/+ slices was more than five times that in *fyn* -/- cultures. In *fyn* -/- cultures, cell death in the presence of ADDLs was at background level. The neuroprotective response was selective; hippocampal cell death evoked by NMDA receptor agonists (Bruce et al., Exper. Neurol., 132, 209-219, 1995; Vornov et al., Neurochem., 56, 996-1006, 1991) was unaffected (not shown). Analysis (ANOVA) using the Tukey multiple comparison gave a value of P < 0.001 for the ADDL *fyn* +/+ data compared to all other conditions.

These results confirm that loss of Fyn kinase protected DG and CA3 hippocampal regions from cell death induced by ADDLs. The results validate that ADDL toxicity is mediated by a mechanism blocked by knockout of Fyn protein tyrosine kinase. These results further suggest that neuroprotective benefits can be obtained by treatments that abrogate the activity of Fyn protein tyrosine kinase or the expression of the gene encoding Fyn protein kinase.

### Example 16: Astrocyte Activation Experiments

To investigate further the potential involvement of signal transduction in ADDL toxicity, the experiments in this example compared the impact on ADDLs on activation of astrocytes.

For these experiments, cortical astrocyte cultures were prepared from neonatal (1-2 day old) Sprague-Dawley rat pups by the method of Levison and McCarthy (Levison et al., In: Banker et al. (Eds.), Culturing Nerve Cells, MIT press, Cambridge, MA, , 309-36, 1991), as previously described (Hu et al., J. Biol. Chem., 271, 2543-2547, 1996). Briefly, cerebral cortex was dissected out, trypsinized, and cells were cultured in α-MEM (Gibco, BRL) containing 10% fetal bovine serum (Hyclone Laboratories Inc., Logan UT) and antibiotics (100 U/mL penicillin, 100 mg/mL streptomycin). After 11 days in culture, cells were trypsinized and replated into 100-mm plates at a density of ∼6 x10⁵ cells/plate and grown until confluent (Hu et al., J. Biol. Chem., 271, 2543-2547, 1996).

Astrocytes were treated with ADDLs prepared according to Example 1, or with Aβ 17-42 (synthesized as per Lambert et al., J. Neurosci. Res., 39, 377-384, 1994; also commercially available). Treatment was done by trypsinizing confluent cultures of astrocytes and plating onto 60 mm tissue culture dishes at a density of 1 x 10⁶ cells/dish (e.g., for RNA analysis and ELISAs), into 4-well chamber slides at 5 x 10⁴ cells /well (e.g., for immunohistochemistry), or into 96-well plates at a density of 5 x 10⁴ cells/well (e.g., for NO assays). After 24 hours of incubation, the cells were washed twice with PBS to remove serum, and the cultures incubated in α-MEM containing N2 supplements for an additional 24 hours before addition of Aβ peptides or control buffer (i.e., buffer containing diluent).

Examination of astrocyte morphology was done by examining cells under a Nikon TMS inverted microscope equipped with a Javelin SmartCam camera, Sony video monitor and color video printer. Typically, four arbitrarily selected microscopic fields (20X magnification) were photographed for each experimental condition. Morphological activation was quantified from the photographs with NIH Image by counting the number of activated cells (defined as a cell with one or more processes at least one cell body in length) in the four fields.

The mRNA levels in the cultures was determined with use of Northern blots and slot blots. This was done by exposing cells to ADDLs or control buffer for 24 hours. After this time, the cells were washed twice with diethylpyrocarbonate (DEPC)-treated PBS, and total RNA was isolated by RNeasy purification mini-columns (Qiagen, Inc., Chatsworth, CA), as recommended by the manufacturer. Typical yields of RNA were 8 to 30 mg of total RNA per dish. For Northern blot analysis, 5 mg total RNA per sample was separated on an agarose-formaldehyde gel, transferred by capillary action to Hybond-N membrane (Amersham, Arlington Heights IL), and UV crosslinked. For slot blot analysis, 200 ng of total RNA per sample was blotted onto Duralon-UV membrane (Stratagene, La Jolla CA) under vacuum, and UV crosslinked. Confirmation of equivalent RNA loadings was done by ethidium bromide staining or by hybridization and normalization with a GAPDH probe.

Probes were generated by restriction enzyme digests of plasmids, and subsequent gel purification of the appropriate fragment. Namely, cDNA fragments were prepared by RT-PCR using total RNA from rat cortical astrocytes. RNA was reverse transcribed with a Superscript II system (GIBCO/ BRL), and PCR was performed on a PTC-100 thermal controller (MJ Research Inc, Watertown, MA) using 35 cycles at the following settings: 52°C for 40 seconds; 72°C for 40 seconds; 96°C for 40 seconds. Primer pairs used to amplify a 447 bp fragment of rat IL-1β were: Forward: 5' GCACCTTCTTTCCCTTCATC 3' [SEQ ID NO:1]. Reverse: 5' TGCTGATGTACCAGTTGGGG 3' [SEQ ID NO:2]. Primer pairs used to amplify a 435 bp fragment of rat GFAP were: Forward: 5' CAQTCCTTGACCTGCGACC 3' [SEQ ID NO:3]. Reverse: 5' GCCTCACATCACATCCTTG 3' [SEQ ID NO:4]. PCR products were cloned into the pCR2.1 vector with the Invitrogen TA cloning kit, and constructs were verified by DNA sequencing. Probes were prepared by *Eco*RI digestion of the vector, followed by gel purification of the appropriate fragments. The plasmids were the rat iNOS cDNA plasmid pAstNOS-4, corresponding to the rat iNOS cDNA bases 3007-3943 (Galea et al., J. Neurosci. Res., 37, 406-414, 1994), and the rat GAPDH cDNA plasmid pTRI-GAPDH (Ambion, Inc., Austin TX).

The probes (25 ng) were labeled with ³²P-dCTP by using a Prime-a-Gene Random-Prime labeling kit (Promega, Madison WI) and separated from unincorporated nucleotides by use of push-columns (Stratagene). Hybridization was done under stringent conditions with QuikHyb solution (Stratagene), using the protocol recommended for stringent hybridization. Briefly, prehybridization was conducted at 68°C for about 30 to 60 minutes, and hybridization was at 68°C for about 60 minutes. Blots were then washed under stringent conditions and exposed to either autoradiography or phosphoimaging plate. Autoradiograms were scanned with a BioRad GS-670 laser scanner, and band density was quantified with Molecular Analyst v2.1 (BioRad, Hercules CA) image analysis software. Phosphoimages were captured on a Storm 840 system (Molecular Dynamics, Sunnyvale CA), and band density was quantified with Image Quant v1.1 (Molecular Dynamics) image analysis software.

For measurement of NO by nitrite assay, cells were incubated with Aβ peptides or control buffer for 48 hours, and then nitrite levels in the conditioned media were measured by the Griess reaction as previously described (Hu et al., J. Biol. Chem., 271, 2543-2547, 1996). When the NOS inhibitor N-nitro-L-arginine methylester (L-name) or the inactive D-name isomer were used, these agents were added to the cultures at the same time as the Aβ.

Results of these experiments are presented in **Figure 13****.** As can be seen in this figure, glia activation increases when astrocytes are incubated with ADDLs, but not when astrocytes are incubated with Aβ 17-42.

These results confirm that ADDLs activate glial cells. It is possible that glial proteins may contribute to neural deficits, for instance, as occur in Alzheimer's Disease, and that some effects of ADDLs may actually be mediated indirectly by activation of glial cells. In particular, glial proteins may facilitate formation of ADDLs, or ADDL-mediated effects that occur downstream of receptor binding. Also, it is known that clusterin is upregulated in the brain of the Alzheimer's diseased subject, and clusterin is made at elevated levels only in glial cells that are activated. Based on this, activation of glial cells by a non-ADDL, non-amyloid stimulus could produce clusterin which in turn might lead to ADDLs, which in turn would damage neurons and cause further activation of glial cells.

Regardless of the mechanism, these results further suggest that neuroprotective benefits can be obtained by treatments that modulate (i.e., increase or decrease) ADDL-mediated glial cell activation. Further, the results suggest that blocking these effects on glial cells, apart from blocking the neuronal effects, may be beneficial.

### Example 17: LTP Assay-ADDLs Disrupt LTP

Long-term potentiation (LTP) is a classic paradigm for synaptic plasticity and a model for memory and learning, faculties that are selectively lost in early stage AD. This example sets forth experiments done to examine the effects of ADDLs on LTP, particularly medial perforant path-granule cell LTP.

**Injections of intact animals:** Mice were anesthesized with urethane and placed in a sterotaxic apparatus. Body temperature was maintained using a heated water jacket pad. The brain surface was exposed through holes in the skull. Bregma and lambda positions for injection into the middle molecular layer of hippocampus are 2 mm posterior to bregma, 1 mm lateral to the midline, and 1.2-1.5 mm ventral to the brain surface. Amyloid β oligomer injections were by nitrogen puff through ∼ 10 nm diameter glass pipettes. Volumes of 20-50 nL of amyloid β oligomer solution (180 nM of amyloid β in phosphate buffered saline, PBS) were given over the course of an hour. Control mice received an equivalent volume of PBS alone. The animal was allowed to rest for varying time periods before the LTP stimulus is given (typically 60 minutes).

**LTP in injected animals:** Experiments follow the paradigm established by Routtenberg and colleagues for LTP in mice (Namgung et al., Brain Research, 689, 85-92, 1995). Perforant path stimulation from the entorhinal cortex was used, with recording from the middle molecular layer and the cell body of the dentate gyrus. A population excitatory postsynaptic potential (pop-EPSP) and a population spike potential (pop-spike) were observed upon electrical stimulation. LTP could be induced in these responses by a stimulus of 3 trains of 400 Hz, 8 x 0.4 ms pulses/train (Namgung et al., Brain Research, 689, 85-92, 1995). Recordings were taken for 2-3 hours after the stimulus (i.e., applied at time 0) to determine if LTP is retained. The animal was then sacrificed immediately, or was allowed to recover for either 1, 3, or 7 days and then sacrificed as above. The brain was cryoprotected with 30% sucrose, and then sectioned (30 µM) with a microtome. Some sections were placed on slides subbed with gelatin and others were analyzed using a free-floating protocol. Immunohistochemistry was used to monitor changes in GAP-43, in PKC subtypes, and in protein phosphorylation of tau (PHF-1), paxillin, and focal adhesion kinase. Wave forms were analyzed by machine as described previously (Colley et al., J. Neurosci., 10, 3353-3360, 1990). A 2-way ANOVA compares changes in spike amplitude between treated and untreated groups.

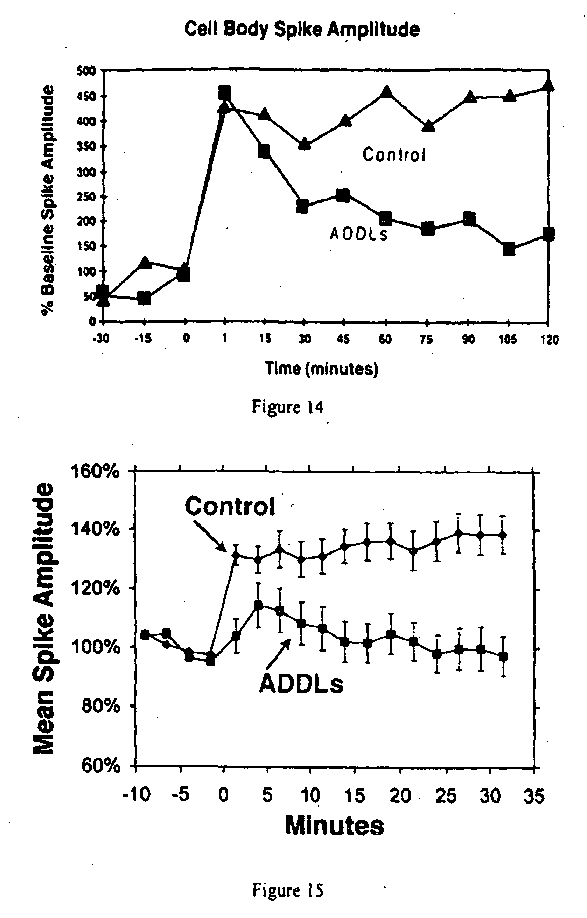

**Figure 14** illustrates the spike amplitude effect of ADDLs in whole animals. As can be clearly seen in this figure, ADDLs block the persistence phase of LTP induced by high frequency electrical stimuli applied to entorhinal cortex and measured as cell body spike amplitude in middle molecular layer of the dentate gyrus.

After the LTP experiment was performed, animals were allowed to recover for various times and then sacrificed using sodium pentobarbitol anesthetic and perfusion with 4% paraformaldehye. For viability studies, times of 3 hours, 24 hours, 3 days, and 7 days were used. The brain was cryoprotected with 30% sucrose and then sectioned (30 µM) with a microtome. Sections were placed on slides subbed with gelatin and stained initially with cresyl violet. Cell loss was measured by counting cell bodies in the dentate gyrus, CA3, CA1, and entorhinal cortex, and correlated with dose and time of exposure of ADDLs. The results of these experiments confirmed that no cell death occurred as of 24 hours following the LTP experiments.

Similarly, the LTP response was examined in hippocampal slices from young adult rats. As can be seen in **Figure 15****,** incubation of rat hippocampal slices with ADDLs prevents LTP well before any overt signs of cell degeneration. Hippocampal slices (n=6) exposed to 500 nM ADDLs for 45 minutes prior showed no potentiation in the population spike 30 minutes after the tetanic stimulation (mean amplitude 99% +/- 7.6), despite a continuing capacity for action potentials. In contrast, LTP was readily induced in slices incubated with vehicle (n=6), with an amplitude of 138% +/-8.1 for the last 10 minutes; this value is comparable to that previously demonstrated in this age group (Trommer et al., Exper. Neurol., 131, 83-92, 1995). Although LTP was absent in ADDL-treated slices, their cells were competent to generate action potentials and showed no signs of degeneration.

These results validate that in both whole animals and tissue slices, the addition of ADDLs results in significant disruption of LTP in less than an hour, prior to any cell degeneration or killing. These experiments thus support that ADDLs exert very early effects, and interference with ADDL formation and/or activity thus can be employed to obtain a therapeutic effect prior to advancement of a disease, disorder, or condition (e.g., Alzheimer's disease) to a stage where cell death results. In other words, these results confirm that decreases in memory occur before neurons die. Interference prior to such cell death thus can be employed to reverse the progression, and potentially restore decreases in memory.

### Example 18: Early Effects of ADDLs in vivo

This example sets forth early effects of ADDLs *in vivo* and the manner in knowledge of such early effects can be manipulated.

The primary symptoms of Alzheimer's disease involve learning and memory deficits. However, the link between behavioral deficits and aggregated amyloid deposits has been difficult to establish. In transgenic mice, overexpressing mutant APP under the control of the platelet-derived growth factor promoter results in the deposition of large amounts of amyloid (Games et al., Nature, 373, 523-527, 1995). By contrast, no behavioral deficits have been reported using this system. Other researchers (i.e., Nalbantoglu et al., Nature, 387, 500-505, 1997 and Holcomb et al., Nat. Med, 4, 97-100, 1998) working with transgenic mice report observing significant behavioral and cognitive deficits that occur well before any significant deposits of aggregated amyloid are observed. These behavioral and cognitive defects include failure to long-term potentiate (Nalbantoglu et al., *supra).* These models collectively suggest that non-deposited forms of amyloid are responsible for the early cognitive and behavioral deficits that occur as a result of induced neuronal malfunction. It is consistent with these models that the novel ADDLs described herein are this non-deposited form of amyloid causing the early cognitive and behavioral defects. In view of this, ADDL modulating compounds according to the invention can be employed in the treatment and/or prevention of these early cognitive and behavioural deficits resulting from ADDL-induced neuronal malfunction, or ADDLs themselves can be applied, for instance, in animal models, to study such induced neuronal malfunction.

Similarly, in elderly humans, cognitive decline and focal memory deficits can occur well before a diagnosis of probable stage I Alzheimer's disease is made (Linn et al., Arch. Neurol., 52, 485-490, 1995). These focal memory deficits may result from induced abberant signaling in neurons, rather than cell death. Other functions, such as higher order writing skills (Snowdon et al., JAMA, 275, 528-532, 1996) also may be affected by abberant neuronal function that occurs long before cell death. It is consistent with what is known regarding these defects, and the information regarding ADDLs provided herein, that ADDLs induce these defects in a manner similar to compromised LTP function such as is induced by ADDLs. Along these lines, ADDL modulating compounds according to the invention can be employed in the treatment and/or prevention of these early cognitive decline and focal memory deficits, and impairment of higher order writing skills, resulting from ADDL formation or activity, or ADDLs themselves can be applied, for instance, in animal models, to study such induced defects. In particular, such studies can be conducted such as is known to those skilled in the art, for instance by comparing treated or placebo-treated age-matched subjects.

### Example 19: Modified Method for Preparing Amyloid β Oligomers

This Example describe an alternative method for making ADDLs that can be employed instead of, for instance, the methods described in Examples 1 and 4.

Amyloid β monomer stock stock solution is made by dissolving the monomer in hexafluoroisoproanol (HFIP), which is subsequently removed by speed vacuum evaporation. The solid peptide is redissolved in dry DMSO at 5 mM to form a DMSO stock solution, and the ADDLs are prepared by diluting 1 µl of the DMSO stock solution into 49 µl of F12 media (serum-free, phenol-red free). The mixture is vortexed and then incubated at 4°C for 24 hours.

### Example 20: Further Gel Studies of Amyloid β Oligomers

This Example describes further gel studies done on amyloid β oligomers.

For gel analysis following preparation of the amyloid β oligomers (i.e., oligomers prepared as described in the prior example), 1 µl of the oligomer solution is added to 4 µl of F12 and 5 µl of tris-tricine loading buffer, and then loaded on a pre-made 16.5% tris-tricine gel (Biorad). Electrophoresis is carried out for 2.25 hours at 100 V. Following electrophoresis, the gel is stained using the Silver Xpress kit (Novex). Alternately, instead of staining the gel, the amyloid β species are transferred from the gel to Hybond-ECL (Amersham) in SDS-containing transfer buffer for 1 hour at 100 V at 4°C. The blot is blocked in TBS-T1 containing 5% milk for 1 hour at room temperature. Following washing in TBS-T1, the blot is incubated with primary antibody (26D6, 1:2000,) for 1.5 hours at room temperature. The 26D6 antibody recognizes the amino terminal region of amyloid β. Following further washing; the blot is incubated with secondary antibody (anti-mouse HRP, 1:3500) for 1.5 hours at room temperature. Following more washing, the blot is incubated in West Pico Supersignal reagents (500 µl of each, supplied by Pierce) and 3 mls of ddH₂O for 5 minutes. Finally, the blot is exposed to film and developed.

Results of such further gel studies are depicted in **Figure 16**, which shows a computer-generated image of a densitometer-scanned 16.5% tris-tricine SDS-polyacrylamide gel (Biorad). The figure confirms a range of oligomeric, soluble ADDLs (labeled "ADDLs"), dimer (labeled "Dimer"), and monomer (labeled "Monomer"). This gel system thus enables visualization of distinct ADDLs comprising from at least 3 monomers (trimer) up to about 24 monomers.

What is not depicted in **Figure 16****,** but which becomes apparent upon comparing gels/Westerns obtained before and alter aggregation is the fact that the tetramer band increases upon aggregation, whereas the pentamer through the 24-mer oligomer species appear only after aggregation. The differences between the silver stained and the immunodetected amounts of the oligomers (especially dimer and tetramer) suggest that the oligomers may represent different conformations obtained upon aggregation.

### Example 21: Further AFM Studies of Amyloid β Oligomers

This Example describes further AFM studies done on amyloid β oligomers.

AFM was done as described in Example 3 except that fractionation on a Superdex 75 column was not performed, and the field was specifically selected such that larger size globules in the field were measured. The analysis is the same from a technical standpoint as that done in Example 3, but in this instance the field that was specifically selected for and examined allows visualization of oligomers that have larger sizes than those that were measured by the section analysis. AFM was carried out using a NanoScope® **III** MultiMode AFM (MMAFM) workstation using TappingMode® (Digital Instruments, Santa Barbara, CA).

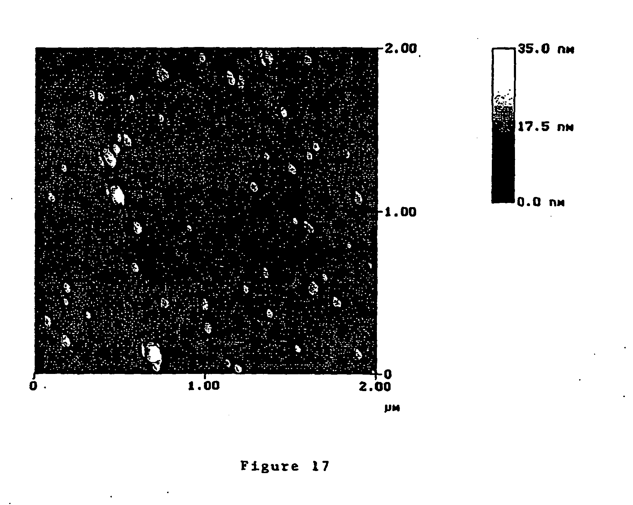

The results of these studies are shown in **Figure 17****,** which is a computer-generated image of an AFM analysis of ADDLs showing various sized structures of different amyloid β oligomers. The adhered structures range in size from 1 to 10.5 nm in z height. Based on this characterization, the structures comprise from 3 to 24 monomeric subunits, consistent with the bands shown on Tris-tricine SDS-PAGE. In separate experiments (not shown) species as high as about 11 nm have been observed.

## Claims

1. An isolated, soluble, non-fibrillar, neurotoxic amyloid β oligomeric structure comprising from 13 to 24 amyloid β proteins, without an exogenous added crosslinking agent, for use in the treatment of Alzheimer's disease, Down's syndrome, mild cognitive impairment, and senile dementia.

2. An isolated, soluble, non-fibrillar, neurotoxic amyloid β oligomeric structure comprising from 13 to 24 amyloid β proteins, without an exogenous added crosslinking agent, for use in the diagnosis of Alzheimer's disease, Down's syndrome, mild cognitive impairment, and senile dementia.

3. The use of an isolated, soluble, non-fibrillar, neurotoxic amyloid β oligomeric structure comprising from 13 to 24 amyloid β proteins, without an exogenous added crosslinking agent, in the preparation of a medicament for the therapeutic and/or prophylactic treatment of Alzheimer's disease, Down's syndrome, mild cognitive impairment, and senile dementia.

## Patentansprüche

1. Isolierte, lösliche, nicht fibrilläre, neurotoxische Amyloid-β-Oligomerstruktur, umfassend von 13 bis 24 Amyloid-β-Proteine, ohne ein exogen zugefügtes Vernetzungsmittel, zur Anwendung in der Behandlung der Alzheimer-Krankheit, des Down-Syndroms, der milden kognitiven Beeinträchtigung und der senilen Demenz.

2. Isolierte, lösliche, nicht fibrilläre, neurotoxische Amyloid-β-Oligomerstruktur, umfassend von 13 bis 24 Amyloid-β-Proteine, ohne ein exogen zugefügten Vernetzungsmittel, zur Verwendung bei der Diagnose der Alzheimer-Krankheit, des Down-Syndroms, der milden kognitiven Beeinträchtigung und der senilen Demenz.

3. Verwendung einer isolierten, löslichen, nicht fibrillären, neurotoxischen Amyloid-β-Oligomerstruktur, umfassend von 13 bis 24 Amyloid-β-Proteine, ohne ein exogen zugefügtes Vernetzungsmittel, bei der Herstellung eines Arzneimittels für die therapeutische und/oder prophylaktische Behandlung der Alzheimer-Krankheit, des Down-Syndroms, der milden kognitiven Beeinträchtigung und der senilen Demenz.

## Revendications

1. Structure oligomère β-amyloïde neurotoxique, non fibrillaire, soluble et isolée, comprenant de 13 à 24 protéines β-amyloïdes, sans agent de réticulation ajouté exogène, destinée à une utilisation dans le traitement de la maladie d'Alzheimer, de la trisomie 21, de la déficience cognitive légère et de la démence sénile.

2. Structure oligomère β-amyloïde neurotoxique, non fibrillaire, soluble et isolée, comprenant de 13 à 24 protéines β-amyloïdes, sans agent de réticulation ajouté exogène, destinée à une utilisation dans le diagnostic de la maladie d'Alzheimer, de la trisomie 21, de la déficience cognitive légère et de la démence sénile.

3. Utilisation d'une structure oligomère β-amyloïde neurotoxique, non fibrillaire, soluble et isolée, comprenant de 13 à 24 protéines β-amyloïdes, sans agent de réticulation ajouté exogène, dans la préparation d'un médicament destiné au traitement thérapeutique et/ou prophylactique de la maladie d'Alzheimer, de la trisomie 21, de la déficience cognitive légère et de la démence sénile.
